# EUROPÄISCHE PATENTANMELDUNG

(11) **EP 1 935 360 A1**
(43) Veröffentlichungstag der Anmeldung: **25.06.2008**
(21) Anmeldenummer: 07123065.0
(22) Anmeldetag: 12.12.2007
(51) Int. Cl.: A61B 17/70, A61B 17/80

(54) **Plattenimplantat, insbesondere für die Anwendung an einer Wirbelsäule, mit einem Schraubenverschlusssystem**

(30) Priorität: 19.12.2006 DE 202006019220 U; 30.01.2007 DE 102007005417
(71) Anmelder: Zrinski AG, 78573 Wurmlingen (DE)
(72) Erfinder: REITZIG, Cliff-Georg, 78604, Weilheim-Rietheim (DE); SCHWEIZER, Barbara, 72172, Sulz am Neckar (DE); ECKHOF, Stephan, 78604, Rietheim-Weilheim (DE)
(74) Vertreter: Muri, Peter

(57) **Zusammenfassung**

Die Erfindung bezieht sich auf ein Plattenimplantat zur Anwendung bei einer Osteosynthese.

Das Plattenimplantat besteht aus einer ersten Plattenkomponente (102; 128), die Durchgangsbohrungen (104) zur Aufnahmen von Befestigungsschrauben (117) und einem Verbindungsmittel (105) aufweist. Die zweite Plattenkomponente (103), die ein Aufnahmemittel (106) zur Aufnahme eines Verbindungsmittels (105) aufweist und Durchgangsbohrungen (115; 129) zur Aufnahme von Befestigungsschrauben (117) zeigt, ist derart angeordnet, dass die beiden Plattenkomponenten (102, 103) bezüglich zueinander gleitend verschiebbar sind. Erfindungsgemäss ist ferner vorgesehen, dass die Befestigungsschrauben (117) aus einem Schraubenschaft und einem Schraubenkopf besteht und der Schraubenschaft (132) und der Schraubenkopf (130) jeweils mit einem Gewinde (131) versehen ist und mindestens eine Plattenkomponente (102,103,128) mindestens eine Durchgangsöffnung (104), bestehend aus mindestens zwei Durchgangsbohrungen (104), aufweist, wobei jede Durchgangsbohrung (104) durch einen Mittelpunkt und durch einen Radius bestimmt ist und die Durchgangsbohrungen zueinander versetzt angeordnet sind und einander schneiden derart, dass sich Schnittlinien oder Schnittflächen ergeben, wobei sich an den Schnittlinien oder Schnittflächen im montierten Zustand mit den Gewinden (131) des Schraubenkopfes (130) zusammenwirken.

Ferner ist als Weiterbildung vorgesehen, dass die beiden Plattenkomponenten (102, 103) durch eine Klemmschraube (118) gegeneinander gepresst werden, jedoch ist beabsichtigt, dass keine Blockade eintritt. Damit kann insbesondere bei Anwendung im Bereich von Wirbelkörpern ein Settling ausgeglichen werden. Zusätzlich können Erweiterungsplatten (128) vorgesehen sein, die das Plattenimplantat in ihrer Länge beliebig verlängern lassen. Sie weisen ebenfalls die Klemmschraube (118) auf.

## Beschreibung

### Technisches Gebiet

Die Erfindung bezieht sich auf ein Plattenimplantat, das in seiner Grundausstattung aus einer ersten und einer zweiten Plattenkomponente besteht. Die erste Plattenkomponente weist Durchgangsbohrungen zur Aufnahme von Befestigungsschrauben und einem Verbindungsmittel auf. Die weitere zweite Plattenkomponente zeigt ein Aufnahmemittel zur Aufnahme eines Verbindungsmittels und Durchgangsbohrungen zur Aufnahme von Befestigungsschrauben. Jede der Plattenkomponenten ist bezüglich der anderen Plattenkomponente in eine Richtung gleitend gelagert und die Plattenkomponenten sind mit einer Vorrichtung versehen, die ihren Gleitlauf zueinander begrenzen.

Ferner umfasst das Plattenimplantat ein Schraubenverschlusssystem, das aus mindestens einer Schraube sowie mindestens einer in der Plattenkomponente vorgesehene Durchgangsbohrung besteht. Die Schraube selbst besteht aus einem Schraubenkopf und einem Schraubenschaft, wobei zumindest der Schraubenkopf mit einem Gewinde versehen ist. Die Schraube ist polyaxial in die Durchgangsbohrung einführbar. Dies bedeutet, dass die Schraube nicht nur senkrecht zur Platte, sondern auch schräg zur Plattenkomponente einführbar und mit der Plattenkomponente verriegelbar ist. Eine kraft- oder eine kraft- und formschlüssige Verbindung zwischen der Schraube und Platte entsteht.

### Definitionen

### Durchgangsbohrung

Als Durchgangsbohrung sind solche Bohrungen definiert, die sich von der Oberfläche einer Plattenkomponente bis zur Unterseite der Plattenkomponente erstrecken. Sie kann zylindrisch oder konisch ausgebildet sein. In der Regel ist sie mit einem Bohrer oder einem Fräser herstellbar.

### Durchgangsöffnung

Als Durchgangsöffnung sind solche Öffnungen definiert, die zur Aufnahme einer Schraube mit Schraubenschaft und Schraubenkopf geeignet sind. In der Durchgangsöffnung geht der Schraubenkopf mit dem übrigen Teil der Plattenkomponente die kraft- und formschlüssige Verbindung ein. Die Durchgangsöffnung entsteht in der Regel erst durch zwei oder mehr Durchgangsbohrungen, die derart zueinander angeordnet sind, dass diese sich schneiden. Zur Herstellung einer Durchgangsöffnung wird zunächst mit einer Zentralbohrung begonnen und ausgehend von dieser werden in vorzugsweise regelmässigen Abständen weitere Durchgangsbohrungen ausgeführt, sodass in Draufsicht eine klee- oder blumen- beziehungsweise blätterartige Konstruktion entsteht.

### Schnittlinie

Schneiden sich mehrere Durchgangsbohrungen, so entstehen im Bereich der Durchgangsöffnung Schnittlinien, die sich über die Dicke der Plattenkomponente erstrecken. Die Schnittlinien erstrecken sich somit in die Tiefe der Durchgangsöffnung. Vorzugsweise sind sie derart ausgebildet, dass sie sich in Richtung der Längsausdehnung der Durchgangsbohrung erstrecken. Die Schnittlinien sind Elemente, die mit dem Schraubenkopf zusammenwirken, um eine kraft- und formschlüssige Verbindung durch plastisches Umformen herbeizuführen.

### Schnittfläche

Aus den zuvor definierten Schnittlinien werden dann Schnittflächen, wenn die einzelnen Durchgangsbohrungen konisch gestaltet sind. Die Schnittflächen sind Elemente, die mit dem Schraubenkopf zusammenwirken, um eine kraft- und formschlüssige Verbindung durch plastisches Umformen herbeizuführen, somit ist eine Schnittfläche dann gegeben, wenn eine flächige Ausbildung in den Raum, hergestellt durch eine Bohrung, zeigt und diese mit einer Schraube zusammenwirken kann. Nachfolgend wird der Einfachheit halber nur von Schnittlinien gesprochen. Sofern nicht anders ausgeführt, wird aber auch darunter die Kombination aus Schnittlinien und Schnittflächen oder nur die Schnittflächen verstanden.

### Stand der Technik

Die Osteosynthese ist ein Verfahren, das zur Versorgung von Knochenbrüchen und anderen Knochenverletzungen dient, bei denen insbesondere Implantate, die meistens aus Metall bestehen, eingesetzt werden. Ziel ist es dabei, die Fixierung der zueinander gehörigen Knochenfragmente in einer normalen Stellung (Reposition) zu fixieren. In der Regel erfolgt die operative Versorgung durch das Anbringen von Metallplatten und Befestigungsschrauben am Knochen oder, besonders bei Frakturen des Schaftes der grossen Röhrenknochen, durch Einbringung von langen Nägeln, die in der Markhöhle entlang der Achse des Knochens gelagert werden.

Insbesondere sind bei der Knochenzusammenführung im Bereich der Halswirbelsäule spezielle Techniken bekannt. Sie werden üblicherweise bei Krankheitsbildern, die auf ein HWS (Halswirbelsäulen) - Syndrom zurückzuführen sind, aber auch bei einem degenerativen Rückgrat eingesetzt. Die bei HWS-Syndromen angewandten Techniken stützen sich darauf, dass operativ die so genannte craniocervikale Instabilität durch Einfügen von Plattenimplantaten verringert beziehungsweise beseitigt wird. Hierzu werden plattenartige Elemente, die mehrere Durchgangsbohrungen zur Aufnahme von Befestigungsschrauben umfassen, auf die dem Körper abgewandten Seite der Wirbelsäule angebracht, sodass mit den Befestigungsschrauben mehrere aufeinander folgende Wirbelkörper miteinander verbunden werden.

Insbesondere stellt sich bei der Befestigung des Plattenimplantats die Schwierigkeit, dass in der Regel der Abstand zwischen den aufeinanderfolgenden Wirbelkörpern oft unterschiedlich ist, sodass eine Vielzahl von unterschiedlichen langen Plattenimplantaten in unterschiedlichen Ausgestaltungen bevorratet werden muss.

Um diesen Umstand zu vermeiden, ist beispielsweise aus der US 5616142 A (YUAN HANSEN A (US); LIN CHIH-1 (US)) 01.04.1997 ein Plattenimplantat bekannt, das aus zwei Plattenkomponenten besteht, die jeweils Aufnahmemittel zur Aufnahme von Verbindungsmitteln einer Zwischenplatte aufweisen. Die Verbindungsmittel selbst sind in den Aufnahmemitteln gleitend gelagert, sodass der Abstand von den beiden Plattenkomponenten zueinander variabel gestaltet werden kann. Die Plattenkomponenten selbst weisen Durchgangsbohrungen auf, in die die Befestigungsschrauben einbringbar sind. Sobald der Abstand der beiden Plattenkomponenten zueinander fixiert ist, wird die Zwischenplatte an den Plattenkomponenten fixiert, sodass ein steifes, nicht mehr zu verschiebendes Plattenimplantat entsteht. Damit ist die Eigenschaft einer einfachen Anpassung an verschiedene Zwischenräume der Knochen beziehungsweise Wirbelkörper gegeben.

Aus der WO 99/004718 (DIMSO DISTRIBUTION MEDICALE DU SUD OUEST) 04.02.1999 ist ein Plattenimplantat, insbesondere eine Knochenplatte für die Anwendung an der Vorderseite einer Wirbelsäule bekannt. Dieses Knochenimplantat zielt darauf ab, insbesondere eine Anwendung an den vorderen Halswirbeln zu bieten. Es besteht aus zwei zueinander gleitenden Plattenelementen, die jeweils Durchgangsbohrungen zur Aufnahme von Befestigungsschrauben aufweisen. Ferner weisen beide ein Verbindungsmittel beziehungsweise ein Aufnahmemittel auf, sodass beide Plattenelemente zueinander gleitend beweglich sind. Aufgrund der Gleitbewegung kann der Abstand der Befestigungsschrauben ausgewählt werden, sodass die Befestigungsschrauben in dem Knochen ausreichend Halt finden. Ist die Position entsprechend gewählt, können die beiden Knochen beziehungsweise Wirbelkörper zusammengedrückt werden, wodurch eine entsprechende Gleitbewegung der beiden Plattenimplantate entsteht. Ist die gewünschte Position erreicht, kann durch Einsetzen einer Fixierschraube die Gleitbewegung unterbrochen und damit vollständig blockiert werden, sodass ein form- und kraftschlüssiges Implantat entsteht.

Schrauben und Plattenkomponente der vorstehenden Art bilden in der Regel ein Fixierungssystem und werden vorwiegend dafür verwendet, Knochen oder Wirbelkörper mechanisch zu stabilisieren. Dieses Fixierungssystem besteht aus mindestens einer Plattenkomponente, die mit mindestens einer Durchgangsbohrung, in der Regel jedoch mehreren Durchgangsbohrungen, versehen ist, wobei die Durchgangsbohrungen zur Aufnahme jeweils einer Befestigungsschraube ausgebildet sind. Die Befestigungsschraube selbst besteht aus einem Schraubenkopf und aus einem Schraubenschaft und weist an dem Schraubenschaft ein Gewinde auf, das dazu geeignet ist, in einen Knochen eingedreht zu werden. Der Schraubenkopf selbst weist ebenfalls ein Gewinde auf, der im montierten Zustand des Fixierungssystems mit der Plattenkomponente in der jeweiligen Durchgangsbohrung beziehungsweise -öffnung zusammenwirkt.

Um zu verhindern, dass diese Befestigungsschrauben sich insbesondere bei Krafteinwirkung lösen, sind aus dem Stand der Technik eine Vielzahl von Mechanismen bekannt, die verhindern, dass ein Loslösen der Schraube und damit ein Loslösen der plattenförmigen Ausbildung möglich ist. Ferner sind Lösungsansätze bekannt, die es ermöglichen, zwischen der Plattenkomponente und der Schraube eine kraftschlüssige Verbindung herzustellen, bei der die Schraube nicht senkrecht zur Plattenkomponente ausgerichtet ist. Eine Schrägstellung einer solchen Schraube ist oft dann gewünscht, wenn beispielsweise aufgrund der Knochenstruktur kein Eindrehen einer Schraube senkrecht zur Plattenkomponente möglich ist.

So sind beispielsweise aus der US 4484570 (SYNTHES LTD (US)) 22.05.1981 sogenannte Expansionskopfschrauben bekannt. Eine einmal in eine Platte eingedrehte Kopfschraube wird mit einem Zusatzschraubelement derart gespreizt, dass durch das Eindrehen der zusätzlichen Schraube eine Verkeilung innerhalb der Platte stattfindet, sodass eine Fixierung der Schraube an der Platte erfolgt.

Eine weitere Ausführungsform aus dem Stand der Technik, wie sie beispielsweise in der US 5954722 (DEPUY ACROMED INC (US)) 29.07.1997 aufgeführt ist, zeigt eine ausgebildete Platte, in die polyaxial eine Schraube eindrehbar ist. Die Polyaxialität hat unter anderem den Vorteil, dass die Einschraubrichtungen nicht mehr durch die Platte selbst vorgegeben sind. Dadurch ist es möglich, je nach Beschaffenheit des Materials, eine Schraube nicht unbedingt senkrecht zu der Plattenlängserstreckung in einen Knochen einzuschrauben. Hierfür ist vorgesehen, dass die Durchgangsbohrungen zusätzlich mit einem kugeligen Element versehen sind, das innerhalb der Durchgangsbohrung zumindest teilweise drehbar gelagert ist. Dieses kugelige Element ist unverlierbar innerhalb der Durchgangsbohrung angeordnet und wirkt mit dem Schraubenkopf der einzudrehenden Schraube zusammen. Je nach Lage orientiert sich das kugelige Element und stellt somit mit dem Schraubenkopf und der Platte eine kraft- und formschlüssige Verbindung her.

Eine weitere polyaxiale Ausführung einer Platte, die mit einer speziell ausgebildeten Schraube zusammenwirkt, ist in der US 2005165400 A (FERNANDEZ ALBERTO A (UY)) 28.07.2005 dargestellt. Das orthopädische Fixationssystem weist eine Platte auf, die ebenfalls ein oder mehrere Durchgangsbohrungen aufweist. Ferner ist eine Schraube vorgesehen, die aus einem Schraubenkopf und einem Schraubenschaft besteht. Der Schraubenkopf ist speziell ausgebildet und weist ein Gewinde auf, das mit zumindest teilweise in den Durchgangsbohrungen vorhandenen Gewinden zusammenwirkt. Um ein Einschrauben schräg (und damit nicht senkrecht) zur Längserstreckung der Platte zu ermöglichen, ist vorgesehen, die Durchgangsbohrung speziell auszubilden. Die Durchgangsbohrungen weisen im Querschnitt eine sanduhrartigen Form auf. Dies bedeutet, dass - in Einschraubrichtung gesehen - sich der Durchmesser der Durchgangsbohrung von einem weiten Durchmesser ausgehend zunächst verjüngt, bis eine definierte Ebene erreicht ist. Von dieser Ebene ausgehend weitet sich der Durchmesser der Durchgangsbohrung wieder auf. Die Durchgangsbohrung, die in dieser Weise ausgebildet ist, weist ein Gewinde auf, das mit dem Gewinde des Schraubenkopfes zusammenwirkt. Das Gewinde ist besonders ausgebildet und als so genanntes Schneidgewinde dargestellt. Dies bedeutet, dass beim Eindrehen des Schraubenkopfes ein mechanischer Schneidevorgang zwischen dem besagten Gewinde und dem Schraubenkopf stattfindet. Um diesen Schneidevorgang zu verstärken und damit ein Verkeilen des Schraubenkopfes innerhalb der Durchgangsbohrung zu erreichen, damit ein ungewolltes Lösen zu einem späteren Zeitpunkt von Platte und Schraube nicht mehr möglich ist, sind innerhalb der Durchgangsbohrung vorzugsweise aus einem anderen Material bestehende Schneidelemente vorgesehen. Diese Schneidelemente sind im Umfang der Durchgangsbohrung eingelassen und führen beim Eindrehen der Schraube in die Durchgangsbohrung eine Verformung herbei. Durch die kugelige Ausgestaltung des Schraubenkopfes ist es möglich, eine Vielzahl von Winkelgraden, die von der Senkrechten zur Längserstreckung der Platte abweichen, zu wählen.

Alternativ hierzu ist vorgesehen, dass ausschliesslich Schneidelemente vorgesehen sind, die mit dem vorgegebenen Gewinde des Schraubenkopfes zusammenwirken. Ein Gewinde innerhalb der Durchgangsbohrung ist nicht vorgesehen. Die Schneideelemente sind jedoch derart angeordnet, dass sie einen Gewindegang bilden.

Aus der DE 202004015912U (AESCULAP AG & CO KG (DE)) 09.12.2004 ist ebenfalls ein Fixationssystem bekannt, das im wesentlichen aus einer Platte und einer Knochenschraube besteht. Die Knochenschraube selbst weist einen mit einer Längsachse definieren Schaft und einen Schraubenkopf aus, welcher mit einer Knochenschraubenaufnahme eine Platte in Eingriff bringbar ist. Zusätzlich ist ein Sicherungselement zum Sichern der Verbindung der Knochenschraube und der Platte vorgesehen, wobei die Knochenschraube von einer Eingriffsstellung, in welcher die Knochenschraube an der Platte gehalten ist, in eine Lösestellung bringbar ist.

Die Durchgangsöffnungen innerhalb der Platte sind vorzugsweise oval gestaltet, sodass eine polyaxiale Aufnahme der Knochenschraube möglich ist. Sie weisen an ihren Wandungen Gewinde auf, die mit dem Gewinde der Knochenschraube beziehungsweise dem Schraubenkopf in Eingriff bringbar sind. Durch Verklemmen der Knochenschraube mit der Platte wird eine Sicherung der Knochenschraube erreicht. Die Herstellung der Durchgangöffnungen wird derart realisiert, dass durch Fräsen eine ovale Durchgangöffnung bereitgestellt wird, die Wandungen aufweist, die senkrecht zur Längserstreckung der Knochenplatte ausgebildet sind. Zusätzlich weisen die Wandungen nur in einem Teilbereich Gewindegänge auf. Die Herstellung daher ist aufwendig und komplex. Die Knochenschraube selbst muss speziell ausgebildet sein und über den speziellen Sicherungsmechanismus verfügen.

Aus der WO 2004/084701 A (SWISS ORTHOPEDIC SOLUTIONS SA (CH); YOUNG ROBERT ALLAN (US)) 07.10.2004 ist eine Knochenplatte bekannt, die eine longitudinale Erstreckung aufweist. Die Knochenplatte selbst weist mehrere Durchgangsöffnungen auf, die durch zwei versetzt zueinander ausgestalteten Durchgangsbohrungen gebildet wird. Die Durchgangsbohrungen sind derart angeordnet, dass deren Mittelpunkt auf der Mittelachse der jeweiligen Knochenplatte angeordnet ist und um einen definierten Abstand voneinander angeordnet sind. Die beiden Durchgangsbohrungen schneiden sich derart, dass in Draufsicht eine achtförmige Ausgestaltung erreicht wird. Das bedeutet, dass zwischen den beiden Durchgangsbohrungen, die die Durchgangsöffnungen bilden, eine Verengung vorgesehen ist. Beide Durchgangsbohrungen weisen im unteren Bereich, dass heisst, dem den Knochen zugewandten Bereich Gewindegänge auf, die zueinander unterschiedlich sind. Auf der dem Knochen abgewandten Seite, dass heisst, auf der Oberseite ist der Öffnungsbereich der Durchgangsbohrung und damit auch der Durchgangsöffnung wesentlich grösser als der wie er im unteren Bereich vorgesehen ist.

Bei besonderen Ausführungsbeispielen sind die im unteren Bereich der Knochenplatte vorgesehenen Gewindegänge in einem Winkel zueinander angeordnet oder aber schräg.

Wie auch beim übrigen Stand der Technik ist es notwendig, die Knochenplatte in unterschiedlichen Bearbeitungszyklen herzustellen. Insbesondere gestaltet es sich als aufwendig, im unteren Bereich der Knochenplatte, dass heisst dem den Knochen zugewandten Bereich unterschiedliche Gewindegänge anzuordnen, die dann mit dem Kopf der Knochenschraube, der ebenfalls ein Gewinde aufweist, zusammenwirken können. Eine freie polyaxiale Anordnung der Knochenschraube ist damit nicht möglich, da sie beim Eindrehen unweigerlich mit dem Gewinde zusammenwirkt und dann die vorgegebene Richtung auch einnimmt.

Auch die DE 20321245U (SYNTHES GMBH (CH)) 14.06.2006 zeigt eine Knochenplatte mit einer dem Knochen zugewandten Unterseite und Oberseite und mehreren die Unterseite mit der Oberseite verbindenden Durchgangsbohrungen, die jeweils eine zentrale Lochachse, eine Innenmantelfläche und einen Gewindegang aufweisen. Die Durchgangsöffnung wird bei einem besonderen Ausführungsbeispiel durch eine zentrale Durchgangsbohrung mit einer die zentrale Durchgangsbohrung am Umfang der Durchgangsbohrung angeordneten Durchgangsbohrungen, die im Winkel von jeweils 90° voneinander beabstandet sind, ausgeführt. Dadurch ist eine polyaxiale Anordnung der jeweiligen Knochenschrauben, deren Kopf sich in die jeweilige Durchgangsbohrung einfügt, gegeben. Die jeweiligen Durchgangsbohrungen weisen wie zuvor bereits beschrieben Gewindegänge auf.

Aus diesem Grund ist auch die Herstellung sehr komplex und die polyaxiale Richtung der jeweiligen Knochenschraube ist aufgrund der entsprechenden Vorgaben der Durchgangsbohrungen nicht frei wählbar.

Zudem ist keine Sicherungsmöglichkeit der jeweiligen Knochenschraube gegeben, sodass ein ungewolltes Lösen jederzeit möglich ist, wodurch die Gefahr gegeben ist, dass die Funktion der jeweiligen Knochenplatte vollständig ausser Funktion gerät.

### Nachteile der Standes der Technik

Unter dem Begriff "Settling" oder "Sinking" (nachstehend stellvertretend "Settling" oder "gesettlet" genannt) ist eine Migration der Knochen beziehungsweise Wirbelkörper zu verstehen. Bevor ein Plattenimplantat angebracht wird, wird zwischen die Wirbelkörper ein Platzhalter eingelegt. Platzhalter können aus einem körperfremden oder einem körpereigenen (beispielsweise Knochen) Material bestehen. Dieser Platzhalter fixiert sich aufgrund der Kräfte, die auf die Wirbelkörper wirken. Doch wegen dauerhafter axialer und vertikaler Belastung verlässt der Platzhalter seine vorgegebene Position. Sinkt der Platzhalter in den Wirbelkörper (aufgrund starker Belastung, Abnutzung, schlechter Qualität des Knochens, etc.), so entsteht zwischen den Wirbelkörpern ein Freiraum, da die Wirbelkörper sich nicht mehr aufeinander zu bewegen können, da ihr Abstand zueinander durch die aus dem Stand der Technik bekannten Plattenimplantate fixiert ist. Die axialen und vertikalen Belastungen werden nun nicht mehr über die Wirbelkörper und den Platzhalter geführt, sondern der Kraftfluss wird über das Plattenimplantat geleitet.

Die dabei entstehenden Kräfte aufgrund dieses Settlingsbeziehungsweise Sinking-Vorgangs sind derart gross, dass die Befestigungsschrauben, die an sich zur Fixierung der Plattenimplantate vorgesehen sind, sich lösen. Dabei geht der Druck, der an sich durch die Befestigungsschrauben auf die Plattenkomponente ausgelöst wird, verloren. Zwischen Schraubenkopf und Plattenkomponente kann ein Abstand entstehen. Dadurch ist das Implantat nicht mehr funktionsfähig und es können weitere Schäden an Knochen und Wirbelkörpern entstehen. Es besteht auch die Gefahr, dass aufgrund der hohen auftretenden Kräfte das Plattenimplantat bricht. Damit kann die eigentliche Funktion des Plattenimplantats vollständig ausser Kraft gesetzt werden.

Somit besteht einer der wesentlichen Nachteile der aus dem Stand der Technik bekannten Plattenimplantate darin, dass das so genannte Settling von den Implantaten nicht berücksichtigt wird.

### Aufgabe der Erfindung

Die Aufgabe der Erfindung ist es, Plattenimplantate gemäss dem Stand der Technik derart weiter zu entwickeln, dass die Befestigungsschrauben in die Plattenkomponenten polyaxial einbringbar und einfach verriegelbar sind.

### Lösung der Erfindung

Die vorliegende Erfindung zielt darauf ab, die Nachteile des Standes der Technik zu vermeiden und ein Plattenimplantat vorzuschlagen, das mindestens aus zwei Plattenkomponenten besteht, die zueinander zumindest für die Zeit der Fixierung auf den Knochen oder Wirbelkörpern gleitend gelagert sind. Zudem wird eine einfache polyaxiale Verriegelung von Befestigungsschrauben und Plattenkomponente erzielt.

### Vorteile der Erfindung

Einer der wesentlichen Vorteile der Erfindung besteht darin, dass trotz der polyaxialen Einsatzmöglichkeit der Schrauben die Plattenkomponente kein Gewinde aufweisen muss. Allein durch die Erstellung der Durchgangsbohrungen in der Plattenkomponente werden Vorsprünge beziehungsweise Schnittlinien erzeugt, die ein insbesondere polyaxiales Einschrauben der Schraube in die Plattenkomponente ermöglichen. Durch die hervorgerufene plastische Verformung wird ebenfalls erreicht, dass ein Lösen der Schraube aus der Plattenkomponente nur noch mit erhöhter Kraftaufwendung möglich ist. Ein ungewolltes Lösen ist nicht möglich.

Vorteilhafterweise sind mindestens drei Durchgangsbohrungen vorgesehen, die zueinander derart angeordnet sind, dass alle drei Durchgangsbohrungen sich schneiden. Dies bedeutet, dass die erste Durchgangsbohrung die zweite Durchgangsbohrung und die dritte Durchgangsbohrung, die zweite Durchgangsbohrung die erste und die dritte und wiederum die dritte Durchgangsbohrung die erste und die zweite Durchgangsbohrung schneidet. Es entstehen dadurch drei Schnittlinien, die sich in die Dicke und damit in die Tiefe der Plattenkomponente erstrecken. In der Mitte entsteht zentrisch einer Durchgangsöffnung, deren Radius durch den Abstand von einer Schnittlinie zum Mittelpunkt der Durchgangsöffnung bestimmt ist.

Eine bevorzugte Ausführungsform sieht vor, dass ausgehend von einer Zentralbohrung ein symmetrischer Aufbau der Durchgangsbohrungen erfolgt. Hierfür sind die Durchgangsbohrungen, die in der Plattenkomponente vorgesehen sind, derart angeordnet, dass deren jeweiligen Mittelpunkte auf einem Kreisbogen ausgehend von dem Mittelpunkt der Zentralbohrung angeordnet sind. Dadurch entsteht eine blumen- , blatt- oder kleeblattähnliche Anordnung der Durchgangsbohrungen um den Mittelpunkt der Zentralbohrung. Die jeweiligen Durchgangsbohrungen schneiden sich. Bei Verwendung von mehreren Durchgangsbohrungen entstehen auch mehrere Vorsprünge beziehungsweise Schnittlinien, die dann wiederum mit dem Schraubenkopf der Schraube zusammenwirken können. Die Zahl der Durchgangsbohrungen ist nicht begrenzt. Die Zahl der Durchgangsbohrungen zur Herbeiführung einer Durchgangsöffnung ist proportional zu den Schnittlinien.

Ein wesentlicher Vorteil ist darin zu sehen, dass keine definierte Ober- und Unterseite der Knochenplatte vorgesehen sein muss. Dies bedeutet, dass die Plattenkomponente unabhängig von ihrer Lage funktionsgerecht verwendet werden kann.

Alle vorgenannten Bohrungen einer besonderen Ausführungsform sind zylindrisch. Dies bedeutet, dass der Durchmesser der Durchgangsbohrungen und damit auch der Durchgangsöffnung über die Dicke der Plattenkomponente gleichbleibend ist.

Die durch das Schneiden der jeweiligen Durchgangsbohrung entstehenden Schnittlinien beziehungsweise Schnittflächen sind derart ausgebildet, dass sie in Draufsicht auf die Plattenkomponente zu dem jeweiligen Zentrum der Durchgangsöffnung hin spitz ausgebildet sind. Sie weisen einen Querschnitt auf, der derart ausgebildet ist, dass dieser von den Wandungen der Durchgangsbohrung breit ausgehend in Richtung Zentrum der Durchgangsöffnung spitz zuläuft. Bei einem Ausführungsbeispiel, bei dem die Durchgangsbohrungen jeweils senkrecht zur Plattenkomponente ausgebildet sind, ist der Querschnitt über die Tiefe der Plattenkomponente der jeweiligen Schnittlinie beziehungsweise Schnittfläche konstant. Dies bedeutet, dass beim Aufsetzen der Schraube auf die erfindungsgemässe Plattenkomponente mit der entsprechenden erfindungsgemässen Ausgestaltung der Durchgangsöffnung bereits mit der ersten Drehung in Eingriff kommt. Um ein besseres Eindrehen der Schraube zu ermöglichen, kann auch vorgesehen sein, dass der die jeweiligen Durchgangsbohrungen und damit auch die gesamte Durchgangsöffnung angesenkt wird. Dadurch kann die Schraube zunächst in die Durchgangsöffnung eingeführt werden, dort entsprechend platziert werden und erst mit einem weiteren Schritt dann in Eingriff mit der jeweiligen Schnittlinie beziehungsweise Schnittfläche gebracht werden.

Werden die Durchgangsbohrungen schräg oder konisch, dass heisst nicht senkrecht zu der Längserstreckung der Plattenkomponente ausgeführt, so entsehen ebenfalls schräge in die Tiefe der Plattenkomponente sich erstreckenden Schnittlinien beziehungsweise Schnittflächen. Werden diese in unterschiedlichen Winkeln noch ausgeführt, so entstehen konische Ausbildungen.

Die Schnittlinien weisen dann an ihren freien Enden eine jeweils sehr geringe Dicke (in Draufsicht gesehen) auf. Dadurch ist es möglich, dass das Gewinde des Schraubenkopfes in diesen Bereichen sich einfach einschneiden kann, da der Widerstand aufgrund der geringen Dicke ebenfalls gering ist. Die in Draufsicht V-förmige Ausbildung der Bereiche der Schnittlinien sind derart, dass mit zunehmenden Abstand von dem freien Ende der Schnittlinie der Querschnitt dicker wird. Sind beispielsweise die Schraubenköpfe konisch gestaltet, so wird mit zunehmenden Eindrehen der Schraube ein grösserer Querschnitt der Schnittlinien beziehungsweise -flächen von dem Gewinde der Schraube durchdrungen. Dadurch findet mit zunehmender Einschraubtiefe eine starke plastische Verformung statt.

Bei einem bevorzugten Ausführungsbeispiel ist vorgesehen, dass nach Herstellung einer zentralen Bohrung die Durchgangsbohrungen in Dickenrichtung der Plattenkomponente nicht zylindrisch ausgestaltet sind, sondern konisch. Die Konizität ist derart ausgestaltet, dass sich die Durchgangsbohrung von der Einschraubeinrichtung zu dem Knochen hin verjüngt. Dies wird vorteilhafterweise durch die Verwendung eines konischen Fräsers erreicht. Vorzugsweise ist deren Symmetrieachse parallel zur Achse der Zentralbohrung und auch senkrecht zur Plattenkomponente selbst. Die Mittelpunkte der Durchgangsbohrungen werden derart ausgewählt, dass deren Radius, der vorzugsweise bei allen Durchgangsbohrungen der gleiche ist, den Durchmesser der Zentralbohrung schneiden, so dass eine Durchgangsöffnung entsteht, die grösser als der Durchmesser der Zentralbohrung ist und an ihrer Aussenwandung eine Vielzahl von konischen Wandungen aufweist. Die einzelnen Wandungen sind konisch ausgebildet und verjüngen sich in Einschraubrichtung. Dadurch entstehen in Einschraubrichtung zunächst Schnittlinien (da der Durchmesser der einzelnen Durchgangsbohrungen gross ist), wohingegen mit zunehmender Tiefe (in Einschraubrichtung) der Durchmesser der Durchgangsbohrung aufgrund der vorgesehenen Konizität sich verringert und daher Schnittflächen zwischen den einzelnen Durchgangsbohrungen entstehen.

Es wurde festgestellt, dass die Anzahl der Durchgangsbohrungen nahezu proportional zur Einschraubkraft der Schraube ist. Dies bedeutet, dass bei einer grösseren Anzahl von Durchgangsbohrungen (beispielsweise 15) die notwendig aufzubringende Einschraubkraft im Vergleich zu einer geringeren Anzahl von Durchgangsbohrungen (beispielsweise 5) geringer ist.

Grundsätzlich ist es vorgesehen, beim Eindrehen der Schraube in die Plattenkomponente mit zunehmender Einschraubtiefe mehr Kraft aufzuwenden. Dies liegt darin begründet, dass zunächst eine Interaktion, vorzugsweise eine Umformung mit den Schnittlinien stattfindet. Die mit der Schraube in Eingriff vorgesehene Linie beziehungsweise Volumen ist gering. Geht die Schnittlinie in eine Schnittfläche über, so wird das von der Schraube umzuformende Volumen grösser und damit muss mehr Kraft aufgebracht werden. Dadurch wird aber auch erreicht, dass eine sichere, nicht lösbare Verbindung zwischen der Plattenkomponente und der Schraube entsteht.

Eine bevorzugte Weiterbildung sieht vor, zunächst die Zentralbohrung mit konischem Querschnitt zu erstellen. Dabei wird eine definierte Konizität gewählt. Die Durchgangsbohrungen, die kleeblattartig in Bezug auf diese Zentralbohrung folgen, weisen ebenfalls eine Konizität auf, aber im Gegensatz zur Zentralbohrung eine andere. Dadurch entstehen im entfernten Einschraubbereich innerhalb der Durchgangsöffnung keine Schnittlinien, sondern Schnittflächen.

Vorzugsweise weisen Schraubenkopf und -schaft denselben Durchmesser auf. Dies bringt den Vorteil mit sich, dass die Schraube einfach herstellbar ist.

Alternativ kann auch vorgesehen sein, dass der Schraubenkopf zylinderförmig ausgebildet ist und eine andere Gewindesteigung als der übrige Schaft aufweist.

Eine weitere Alternative kann vorsehen, dass der Schraubenkopf konisch ausgebildet ist und so sich an die Ausgestaltung der Durchgangsbohrung anpasst.

Der Schraubenschaft selbst kann auch konisch ausgebildet sind, um so den Kraftaufwand, insbesondere beim Eindrehen der Schraube in den Knochen, zu verringern.

Eine weitere alternative Ausführungsform sieht vor, den Schraubenkopf zumindest teilweise kugelig auszugestalten. Dadurch kann erreicht werden, dass ein grösserer Winkel, abweichend von der Senkrechten zur Plattenkomponente erreicht wird, indem die Schraube noch innerhalb der Plattenkomponente schräg (polyaxial) eingedreht werden kann.

Somit ist ein System vorgestellt worden, das ausschliesslich aus zwei Bauteilen besteht. Es ist nicht notwendig, zusätzliche Spreizelemente oder Unterlegelemente oder sonstige Materialien zu verwenden. Schraube und Plattenkomponente können vorzugsweise aus demselben Material hergestellt werden. Um die plastische Verformung, insbesondere im Bereich der Vorsprünge, noch zu verbessern, kann vorgesehen sein die Plattenkomponente aus einem weicheren Material herzustellen als die Schraube selbst. Da die auftretenden Zug- und Schubkräfte gerade bei der Anwendung von Plattenimplantaten im Wirbelsäulenbereich gross sind, eignet sich das erfingungsgemässe Schraubenverschlusssystem besonders. Es verhindert ein ungewolltes Lösen der Plattenkomponente auf sehr einfache Weise.

Einer der wesentlichen Vorteile des Systems liegt darin, dass die Plattenkomponente selbst mit wenig Aufwand herzustellen ist. Die einfachste Ausführung besteht darin, drei Durchgangsbohrungen vorzusehen, die in einer bestimmten Anordnung zueinander auszubilden sind, so dass eine Durchgangsöffnung entsteht, in die die Schraube einführbar ist.

Die bevorzugte Ausbildung sieht vor, eine Zentralbohrung in der Plattenkomponente vorzusehen, die von Durchgangsbohrungen geschnitten wird, wobei die Durchgangsbohrungen mit einem Kegelfräser hergestellt werden, der sich in Einschraubtiefe verjüngt. Die Anzahl der Durchgangsbohrungen sollte grösser als 5 sein. Eine bevorzugte Anzahl ist 15.

Eine weitere vorteilhafte Ausbildung kann vorsehen, dass die Schnittlinien oder/und Schnittflächen gehärtet sind und so gegenüber dem übrigen Material der Plattenkomponente eine härtere Eigenschaft darstellen. Eine solche Härtung ist beispielsweise mit Laser durchführbar.

Ferner geht die Erfindung in einer besonderen Ausführung einen vollständig anderen Weg, als den, den der Stand der Technik vorschlägt. Aufgrund der aufgeführten Klemmwirkung zwischen den Plattenkomponenten wird im Gegensatz zum Stand der Technik, bei dem eine vollständige Blockade der Gleitbewegung der Plattenkomponenten nach der Justierung erzielt wird, erreicht, dass durch das Settling eine Verschiebung der beiden Plattenkomponenten zueinander entgegen einer definierten Klemmkraft möglich ist. Die Klemmkraft wird durch das Anzugsmoment der Klemmschraube, aber auch durch die Klemmwirkung, die der Schraubenkopf der Klemmschraube durch Zusammenwirken mit der Längsbohrung erzielt, hervorgerufen.

Damit können sich die Knochen beziehungsweise Wirbelkörper aufeinander zubewegen, wenn der Platzhalter nicht mehr an dem an sich gedachten beziehungsweise vorgesehenen Platz vorhanden ist. Dadurch verbleibt der Kraftfluss zwischen den Knochen beziehungsweise Wirbelkörpern und verläuft nicht über das Plattenimplantat selbst, wodurch gewährleistet ist, dass die Befestigungsschrauben nicht mit derart hohen Kräften belastet werden, dass eine Lockerung oder sogar eine Loslösung aus dem Knochen beziehungsweise aus den Wirbelkörpern erfolgen kann.

Einer der weiteren Vorteile der Erfindung ist es, dass das Plattenimplantat hinsichtlich seiner Führungselemente derart ausgestaltet ist, dass bei Verschiebungen des Plattenimplantats Teile dieses Implantats nicht über den Wirbelkörper beziehungsweise Knochen gleiten. Dadurch werden Irritationen der Knochenhaut vorteilhafterweise vermieden.

Einer der Vorteile der Erfindung besteht darin, dass das Plattenimplantat aus beliebigem Material herstellbar ist. So ist es zum Beispiel denkbar, dass es neben Titan auch aus spritzgussfähigem Kunststoff herstellbar ist.

Die Geometrie der Plattenkomponenten ist sehr klein gewählt. Dies bringt den Vorteil mit sich, dass diese einzelnen Plattenkomponenten exakt auf die jeweiligen Wirbelkörper aufgesetzt werden können, ohne dass diese einen Überstand (über die Geometrie des Wirbelkörpers hinaus) bilden. Denn aus dem Stand der Technik ist bekannt, dass insbesondere zu lang gewählte Plattenelemente, die über in den Plattenelementen vorgesehene Langlöcher mit Schrauben befestigt werden, sich beim Settlingvorgang in den benachbarten Wirbelkörper oder in die Bandscheiben schieben und so Sekundärschäden hervorrufen.

Vorzugsweise sind Plattenbreiten bis kleiner 22 mm und Plattendicken kleiner als 2,5 mm vorgesehen.

Zudem bietet das Plattenimplantat der vorliegenden Erfindung gegenüber dem Stand der Technik den Vorteil, dass es vor der Adaption auf Knochen oder Wirbelkörper bereits zusammengefügt werden kann und Fixiermittel umfasst, mittels denen eine einfache und vorläufige Fixierung auf Knochen und Wirbelkörpern möglich ist, um so eine Anpassung der einzelnen Abstände und das Finden der optimalen Anbringung der Befestigungsschrauben auf einfache Art und Weise zu ermöglichen.

Ein weiterer Vorteil der Erfindung besteht darin, dass das Plattenimplantat als Bausatz vorliegt. Unter Zugrundelegung der jeweiligen Plattenkomponenten kann durch Einfügen unterschiedlicher Anzahl von Erweiterungsplatten die Länge des Plattenimplantats frei je nach Anwendungsfall gewählt werden. Dabei bleibt weiterhin die eingeschränkte Gleitbewegung zwischen jeder einzelnen Plattenkomponente gewährleistet. Die einzelnen Plattenkomponenten und die Erweiterungsplatten weisen trotzdem eine sehr kleine Geometrie auf, sodass die zuvor genannten Sekundärschäden vermieden werden.

Die Erweiterungsplatten bestehen vorzugsweise ebenfalls aus einem plattenartigen Grundkörper, der Durchgangsbohrungen für die Aufnahme von Befestigungsschrauben aufweist. Ferner sind Aufnahmemittel zur Aufnahme eines Verbindungsmittels und ein Verbindungsmittel selbst vorgesehen. Längsbohrungen zur Aufnahme einer weiteren Klemmschraube und zur Begrenzung des Gleitlaufs sind ebenfalls vorgesehen. Damit kann aus mindestens drei unterschiedlichen Plattenkomponenten ein Plattenimplantat definierter Länge hergestellt werden. Der Anwender kann unmittelbar vor Ort entscheiden, welche Länge notwendig ist. Diese kann durch die Anzahl der Erweiterungsplatten bestimmt werden. Dadurch entfällt eine erheblich kostenaufwendige Bevorratung von unterschiedlichen Plattenimplantaten unterschiedlicher Länge.

Die Plattenimplantate selbst weisen sowohl in ihrer Längs- als auch in ihrer Querstreckung Krümmungen auf. Zusätzlich sind sie aufgrund ihrer Dicke entsprechend an die Kontur der Knochen beziehungsweise der Wirbelkörper anpassbar, indem sie von dem Anwender entsprechend zurechtgebogen werden können.

Dabei geht aber die Eigenschaft nicht verloren, dass die Verbindungsmittel in den Aufnahmemitteln frei gleiten können, damit das entsprechende Settling nachvollzogen werden kann.

Aufgrund der Ausgestaltung der einzelnen Plattenkomponenten beziehungsweise der Erweiterungsplatten ist es für den Anwender möglich, auf sehr einfache Art und Weise die Reihenfolge zur Montage des Plattenimplantats einzuhalten, da es aufgrund der einfachen optischen Struktur von Plattenkomponenten und Erweiterungsplatten ohne weiteres selbsterklärend erfasst werden kann, welche Plattenelemente zusammenzufügen sind.

Um diesen Vorgang weiter zu unterstützen, sind die einzelnen Plattenelemente farbig ausgestaltet, sodass der Anwender unmittelbar die Reihenfolge von erster Plattenkomponente, mindestens einer Erweiterungsplatte sowie der weiteren Plattenkomponente erfassen und umsetzen kann. Durch ein einfaches Stecksystem können die einzelnen Plattenelemente bereits funktionell miteinander zusammengefügt werden, sodass einzelne Gliederelemente entstehen. Die Funktionsfähigkeit kann bereits beim ersten Einstecken geprüft werden.

Durch Vorfixieren der Klemmschraube (noch nicht vollständiges Aufbringen eines Anziehmoments auf die Klemmschraube) bleibt zwar die Gleitbewegung der Plattenelemente untereinander erhalten, ist jedoch in den entsprechenden Bewegungsfreiheiten bereits eingeschränkt. Die vorgesehene Klemmkraft, die zwischen den Plattenelementen wirken soll, wird erst durch ein weiteres Hineindrehen der Klemmschraube in das Verbindungsmittel bewirkt.

Zusätzliche Bohrungen in den jeweiligen Plattenelementen können dafür vorgesehen werden, auf den Knochen beziehungsweise Wirbelkörpern die Plattenelemente vorzufixieren, damit ein Anbohren der Knochen zur Durchführung der Befestigung des Befestigungsschrauben möglich ist.

Weitere vorteilhafte Ausgestaltungen gehen aus der nachfolgenden Beschreibung, den Ansprüchen sowie den Zeichnungen hervor.

### Kurze Beschreibung der Abbildungen der Zeichnungen

Es zeigen:

Fig. 1 ein erstes Ausführungsbeispiel einer schematische Darstellung einer Durchgangsöffnung einer Plattenkomponente als Teil des erfindungsgemässen Schraubenverschlusssystem;

Fig. 2 ein zweites Ausführungsbeispiel einer schematischen Darstellung einer Durchgangsöffnung einer Plattenkomponente als Teil des erfindungsgemässen Schraubenverschlusssystem;

Fig. 3 ein drittes Ausführungsbeispiel einer schematische Darstellung einer Durchgangsöffnung einer Plattenkomponente als Teil des erfindungsgemässen Schraubenverschlusssystem;

Fig. 4 eine schematische Darstellung des orthopädischen Schraubenverschlusssystems im Schnitt, wobei die Schraube bereits in die Plattenkomponente eingedreht ist;

Fig. 5A eine perspektivische Ansicht auf das Schraubenverschlusssystem gemäss Fig. 4;

Fig. 5B eine perspektivische Ansicht auf die Plattenkomponente ohne Schrauben gemäss Fig. 5A;

Fig. 6 eine perspektivische Ansicht auf eine Plattenkomponente mit einer Vielzahl Durchgangsöffnungen, wobei eine Durchgangsöffnung mit einer Schraube belegt ist;

Fig. 7 eine schematische Darstellung einer in einer Plattenkomponente befindlichen Durchgangsöffnung, bestehend aus mehreren zylindrischen Durchgangsbohrungen, zur Erläuterung der Herstellung;

Fig. 8 eine weitere schematische Darstellung einer in einer Plattenkomponente befindlichen Durchgangsöffnung, bestehend aus mehreren konischen Durchgangsbohrungen;

Fig. 9 eine andere schematische Darstellung eines anderen Ausführungsbeispiels einer in einer Plattenkomponente befindlichen Durchgangsöffnung, bestehend aus mehreren konischen Durchgangsbohrungen.

Fig. 10 zeigt eine perspektivische Ansicht auf das Plattenimplantat, bestehend aus einer ersten Plattenkomponente und einer zweiten Plattenkomponente, wobei die beiden Plattenkomponenten nicht miteinander verbunden sind;

Fig. 11 eine perspektivische Ansicht auf die in Fig. 10 dargestellten Plattenkomponenten jedoch mit Darstellungen der Befestigungsschrauben und der erfindungsgemässen Klemmschraube;

Fig. 12 eine perspektivische Ansicht auf ein Plattenimplantat, bestehend aus zwei Plattenkomponenten mit noch nicht fixierter Klemmschraube;

Fig. 13 eine perspektivische Ansicht auf das montierte Plattenimplantat gemäss Fig. 12, jedoch mit montierter Klemmschraube;

Fig. 14 eine Draufsicht auf ein montiertes Plattenimplantat bestehend aus zwei Plattenkomponenten im Bereich der Halswirbelsäule im nicht-gesettleten Zustand;

Fig. 15 eine Draufsicht auf ein montiertes Plattenimplantat, bestehend aus zwei Plattenkomponenten im Bereich der Halswirbelsäule im gesettleten Zustand;

Fig. 16 drei perspektivische Ansichten auf ein Erweiterungselement;

Fig. 17 eine perspektivische Ansicht eines Ausführungsbeispiels eines Plattenimplantats bestehend aus zwei Plattenkomponenten sowie einer Erweiterungsplatte;

Fig. 18 eine perspektivische Darstellung der Plattenkomponenten mit der Erweiterungsplatte zusammen mit Befestigungsschrauben und Klemmschrauben;

Fig. 19 eine perspektivische Ansicht auf ein zusammengefügtes Plattenimplantat bestehend aus zwei Plattenkomponenten und einer Erweiterungsplatte mit noch nicht eingefügten Klemmschrauben;

Fig. 20 eine perspektivische Ansicht auf ein zusammengefügtes Plattenimplantat bestehend aus zwei Plattenkomponenten und einer Erweiterungsplatte mit eingefügten Klemmschrauben;

Fig. 21 eine Draufsicht auf ein montiertes Plattenimplantat bestehend aus zwei Plattenkomponenten und drei Erweiterungsplatten, montiert im Bereich eines Halswirbels im nicht gesettleten Zustand;

Fig. 22 zeigt eine andere perspektivische Ansicht auf ein montiertes Plattenimplantat, bestehend aus zwei Plattenkomponenten und drei Erweiterungsplatten, montiert im Bereich eines Halswirbels im nicht-gesettleten Zustand;

Fig. 23 eine perspektivische Ansicht auf die Klemmschraube;

Fig. 24 einen Schnitt durch ein Plattenimplantat im Bereich der Klemmschraube;

Fig. 25 eine vergrösserte Darstellung des Klemmbereichs gemäss Fig. 23;

Fig. 26 eine weitere vergrösserte Darstellung des Klemmbereichs im Bereich der Längsbohrung.

### Beschreibung von Ausführungsbeispielen

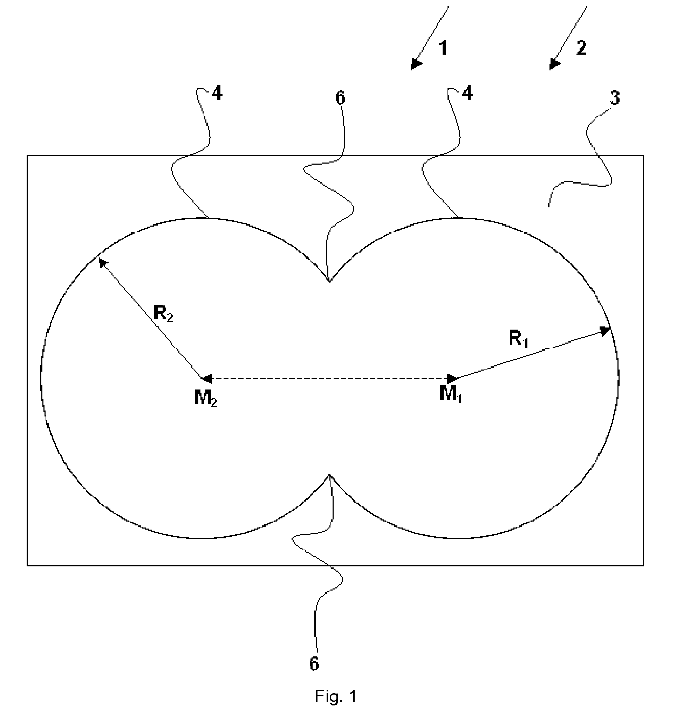

In den Fig. 1 bis 3 ist eine Auswahl von möglichen Durchgangsöffnungen 1 für ein orthopädisches Schraubenverschlusssystem 2 dargestellt. Zur Herstellung einer solchen erfindungsgemässen Durchgangsöffnung 1, angeordnet auf einer Plattenkomponente 3, werden mindestens zwei Durchgangsbohrungen 4 erzeugt. Jede Durchgangsbohrung 4 ist durch einen Radius R₁, R₂ und einen Mittelpunkt M₁, M₂ bestimmt.

Gemäss einem ersten Ausführungsbeispiel, wie es in Fig. 1 dargestellt ist, besteht die Durchgangsöffnung 1 aus zwei Durchgangsbohrungen 4, deren beiden Mittelpunkte M₁, M₂ in einem Abstand 5 zueinander angeordnet sind. Der Abstand 5 ist derart zu wählen, dass die beiden Radien R₁, R₂ der Durchgangsbohrungen 4 sich schneiden. Dadurch entstehen Schnittlinien 6, die sich in die Tiefe (Dicke) der Plattenkomponente 3 erstrecken. Die Schnittlinien 6 sind derart ausgebildet, dass sie zum freien Raum der Durchgangsöffnung 1 hin spitz ausgebildet sind und sich von hier ausgehend mit zunehmender Entfernung eine dickere Wandstärke aufweisen. Dadurch ist es möglich, dass die so gebildeten freien Enden durch ein Eindrehen einer Schraube plastisch verformbar sind.

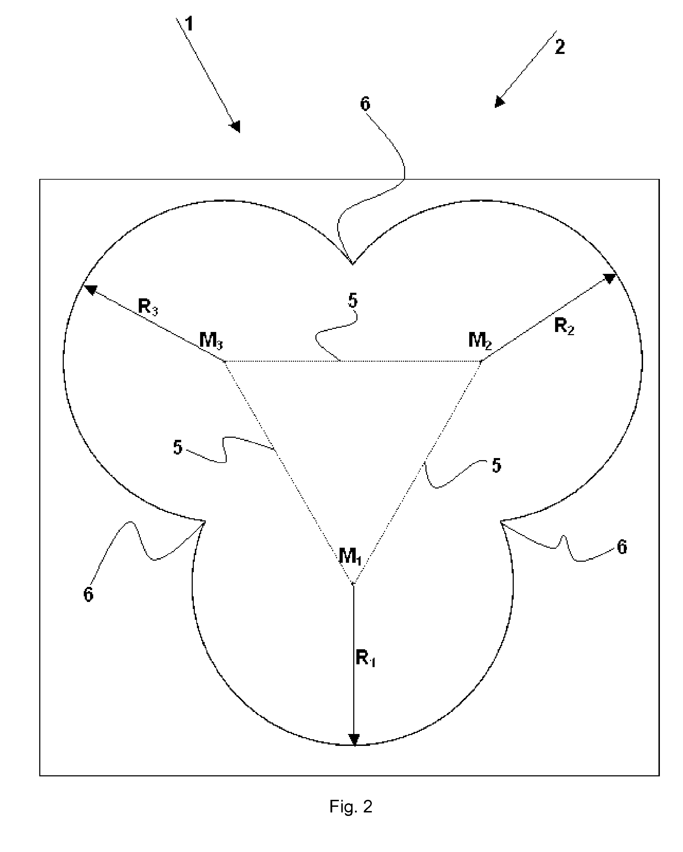

Gemäss einem weiteren Ausführungsbeispiel, wie es in Fig. 2 dargestellt ist, besteht die Durchgangsöffnung 1 aus drei Durchgangsbohrungen 4, deren Mittelpunkte M₁, M₂, M₃ jeweils in einem Abstand 5 voneinander angeordnet sind. Der jeweilige Abstand 5 ist derart zu wählen, dass die drei Radien R₁, R₂, R₃ der Durchgangsbohrungen 4 sich schneiden. Dadurch entstehen Schnittlinien 6, die sich in die Tiefe (Dicke) der Plattenkomponente 3 erstrecken.

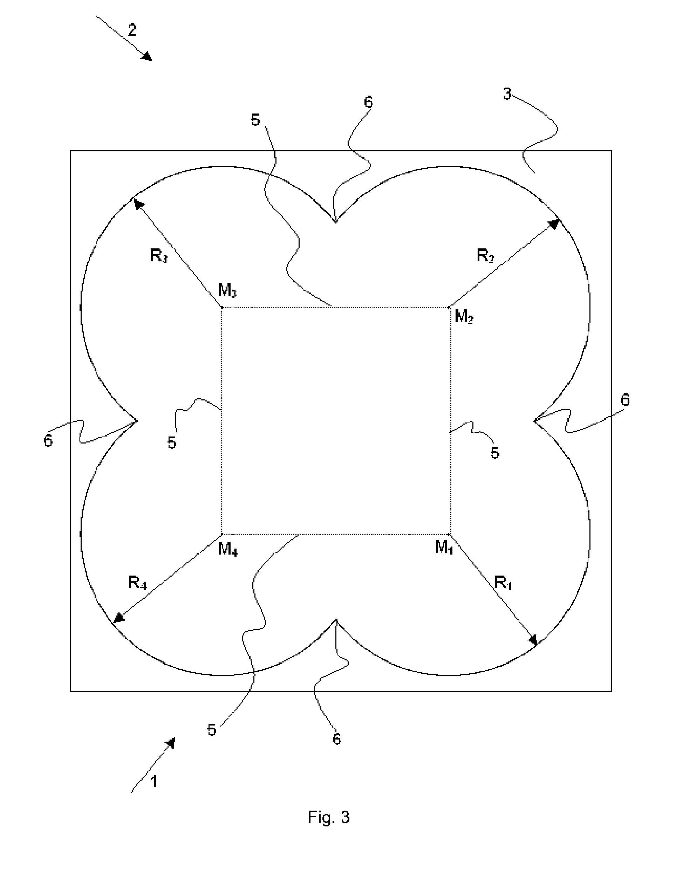

Gemäss einem weiteren Ausführungsbeispiel, wie es in Fig. 3 dargestellt ist, besteht die Durchgangsöffnung 1 aus vier Durchgangsbohrungen 4, deren Mittelpunkte M₁, M₂, M₃, M₄ jeweils in einem Abstand 5 voneinander angeordnet sind. Der jeweilige Abstand 5 ist derart zu wählen, dass die vier Radien R₁, R₂, R₃, R₄ der Durchgangsbohrungen 4 sich schneiden. Dadurch entstehen Schnittlinien 6, die sich in die Tiefe (Dicke) der Plattenkomponente 3 erstrecken.

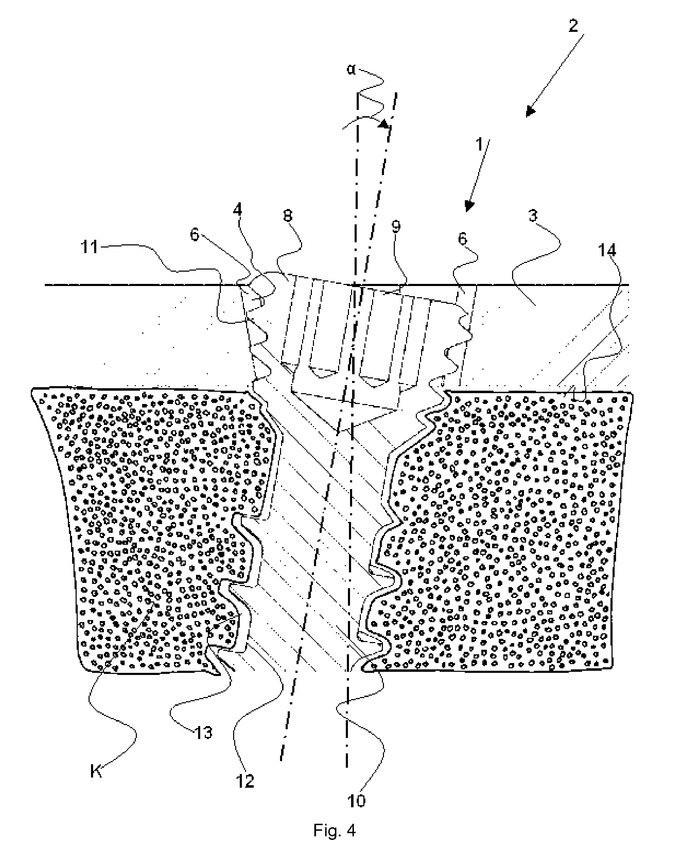

In den Fig. 4, 5A, 5B und 6 ist das orthopädische Schraubenverschlusssystem 2 dargestellt. Es besteht aus einer Schraube 8 und der Plattenkomponenten 3, wie sie beispielsweise in den Fig. 1 bis 3 dargestellt ist. Die Plattenkomponente 3 dient zur Fixierung beispielsweise einer Fraktur. Die Schraube 8 selbst ist in einen Knochen K eingedreht. Die Plattenkomponente 3 liegt mit ihrer Unterseite 14 auf der Aussenfläche des Knochens K auf (Fig. 4). Die Durchgangsöffnung 1, wie sie in den Fig. 1-3 exemplarisch dargestellt ist, dient zur Aufnahme der Schraube 8, die aus einem Schraubenkopf 9 und einem Schraubenschaft 10 besteht. Sowohl Schraubenkopf 9 als auch der Schraubenschaft 10 weisen jeweils ein Gewinde auf. Das Gewinde 11 des Schraubenkopfs 8 wirkt mit den Schnittlinien 6 zusammen. Das Gewinde 12 des Schraubenschafts 10 wirkt mit dem Knochen K zusammen. Im Knochen K selbst ist vorzugsweise bereits eine Bohrung 13 vorgesehen, deren lichte Weite geringer ist, als der Durchmesser des Schraubenschafts 10. Aufgrund dessen, dass die lichte Weite der Durchgangsöffnung 1 grösser ist als die des Durchmessers des Schraubenkopfs 9, ist es möglich, so wie auch in Fig. 4 dargestellt, die Schraube 8 nicht senkrecht zur Plattenkomponente 3, sondern geneigt in einem beliebigen Winkel α anzuordnen.

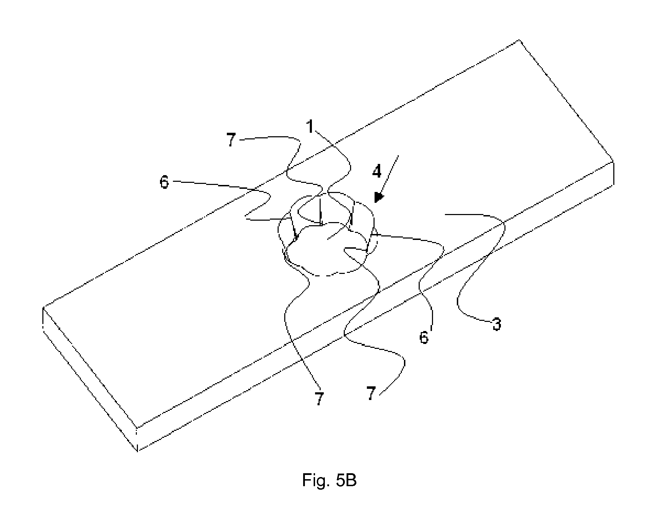

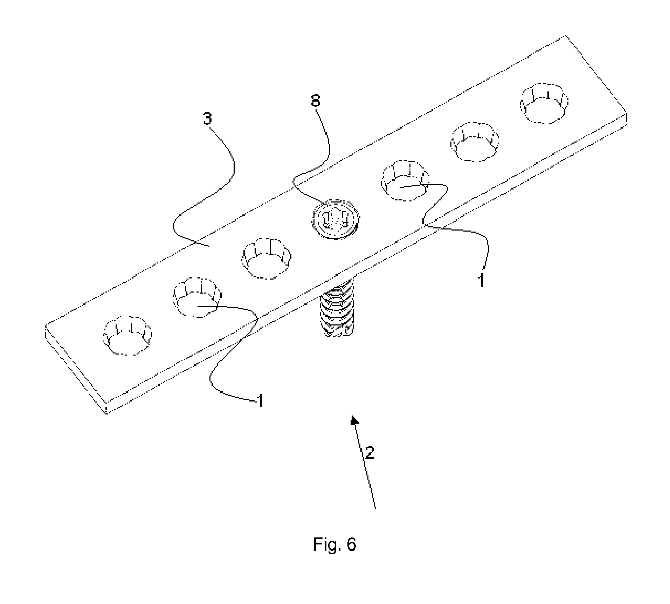

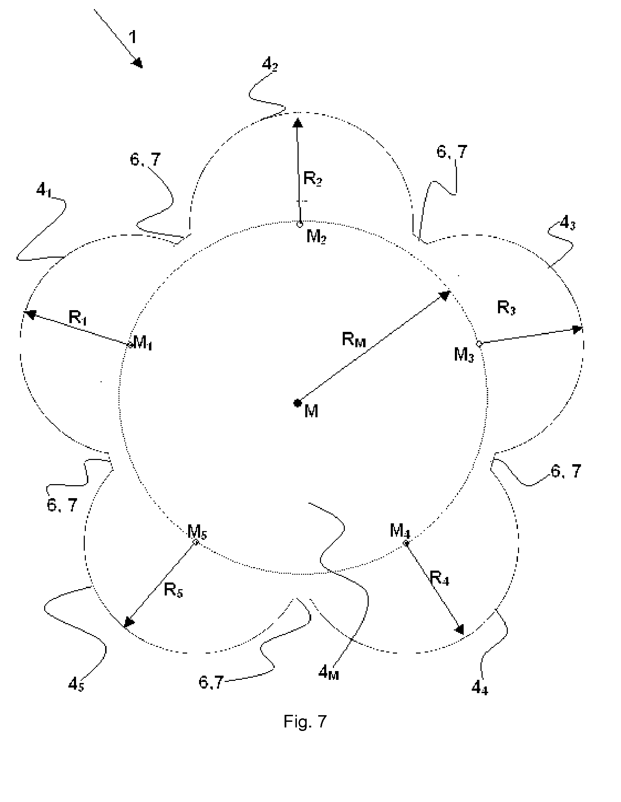

In Fig. 7 ist schematisch dargestellt, wie eine Durchgangsöffnung 1, wie sie in Fig. 5B und Fig. 6 dargestellt ist, konstruiert wird. Zunächst wird der Mittelpunkt M der Durchgangsöffnung 1 festgelegt. Anschliessend wird ein Radius RM festgelegt. Dieser dient dazu, dass alle auf dem Kreis mit dem Radius RM liegenden weiteren Mittelpunkte M₁, M₂, M₃, M₄, M₅ den gleichen Abstand zum Mittelpunkt R_{M} aufweisen. Anschliessend wird der Kreis mit dem Mittelpunkt R_{M} derart aufgeteilt, dass eine beliebige Anzahl von weiteren Mittelpunkten für weitere Kreise im gleichen Abstand zueinander angeordnet sind. Hier in Fig. 6 wird der Kreis derart aufgeteilt, dass die Mittelpunkte M₁, M₂, M₃, M₄, M₅ den gleichen Abstand zueinander haben.

Zunächst ist eine Zentralbohrung 4_{M} mit dem Mittelpunkt M und dem Radius R_{M} zu erzeugen. Die Zentralbohrung 4M bei dem dargestellten Ausführungsbeispiel ist zylinderförmig und gleichförmig über die gesamte Dicke der Plattenkomponente 3. Anschliessend wird die erste Durchgangsbohrung 4₁ mit einem Mittelpunkt M₁ und einem Radius R₁ erzeugt. Sobald die Bohrung erstellt ist, ist eine zylinderförmige Durchgangsbohrung 4₁ entstanden. Bei den Punkten M₂ bis M₅ wird dann wiederum eine Durchgangsbohrung 4₂...4₅ angesetzt und zwar mit dem Radien R₂...R₅, wobei die Radien R₁...R₅ identisch sind. Dadurch entsteht die kleeblättrige Form mit den jeweiligen Schnittlinien 6, die mit dem Schraubenkopfes 9 zusammenwirken. Diese Ausführungsform bietet die Möglichkeit, nach Herstellung einer Zentralbohrung 4M eine Vielzahl von Durchgangsbohrungen 4ₓ mit Radius Rₓ und Mittelpunkt Mₓ bereitzustellen. Dabei ist x Element der natürlichen Zahlen von 1 bis unendlich.

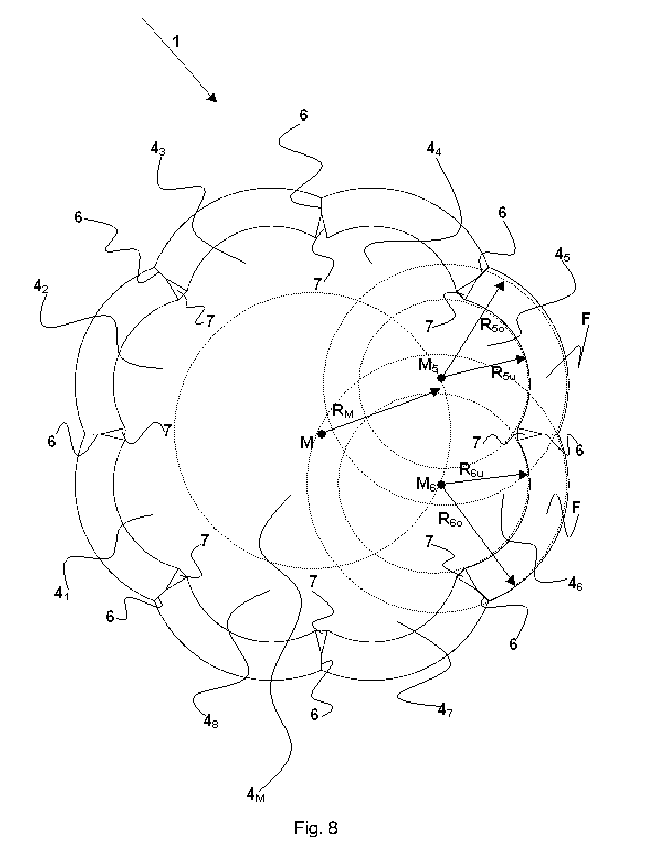

Fig. 8 zeigt eine weitere Ausbildungsform der Ausführung gemäss Fig. 7. Zunächst ist eine Zentralbohrung 4_{M} mit dem Mittelpunkt M und dem Radius R_{M} zu erzeugen. Die Zentralbohrung 4_{M} bei dem dargestellten Ausführungsbeispiel kann konisch sein, wobei diese derart ausgestaltet sein kann, dass sich diese über die Dicke der Plattenkomponente 3 ausgehend von der Oberseite der Plattenkomponente verjüngt.. Die definierte Konizität, dass heisst eine Veränderung des Durchmessers des Konus in Abhängigkeit von der Dicke der Plattenkomponente, ist derart gewählt, dass sich der Durchmesser in Schraubrichtung der Schraube verjüngt. Aufgrund dessen, dass die weiteren Durchgangsbohrungen 4ₓ die Zentralbohrung 4_{M} zumindest nahezu vollständig schneiden, reicht es aus, die Zentralbohrung 4_{M} zylindrisch auszugestalten. Nur dann, wenn der Radius der Zentralbohrung 4_{M} derart gewählt ist, dass dieser dem Radus der Durchgangsöffnung 1 entspricht, ist zur Herstellung eines Ausfühungsbeispiels des Schraubenverschlusssystems die Zentralbohrung konisch zu gestalten.

Anschliessend wird die erste Durchgangsbohrung 4₁ mit einem Mittelpunkt M₁ und einem Radius R₁ erzeugt. Die Durchgangsbohrung 4₁ bei dem dargestellten Ausführungsbeispiel ist konisch ausgestaltet und verjüngt sich über die Dicke der Plattenkomponente 3. Die Konizität, dass heisst eine Veränderung des Durchmessers des Konus in Abhängigkeit von der Dicke der Plattenkomponente, ist derart gewählt, dass sich der Durchmesser in Schraubrichtung der Schraube verjüngt. Sobald die Bohrung erstellt ist, ist eine konische Durchgangsbohrung 4₁ entstanden. Bei den Mittelpunkten M₂ bis M₅ wird dann wiederum eine Durchgangsbohrung 4₂...4₅ angesetzt und zwar mit dem Radien R₂...R₅, wobei die Radien R₁...R₅ und die Konizitäten identisch sind. Dadurch entsteht die in Draufsicht kleeblättrige Form mit den jeweiligen Schnittlinien 6, die mit dem Schraubenkopfes 9 zusammenwirken. Zusätzlich entstehen aufgrund der Konizitäten Durchgangsbohrungen 4ₓ bevorzugt in Verlängerung der Schnittlinie 6 zusätzliche Schnittflächen 7. Dies bedeutet, dass die Schnittlinien 6 mit zunehmender Tiefe in Schnittflächen 7 übergehen. Die Ausbildung des für die Herstellung der Durchgangsbohrung 4ₓ notwendigen konischen Fräsers ist ebenfalls dargestellt. Bei den dargestellten Durchgangsbohrungen 4₅ und 4₆ ist ausgehend von den Mittelpunkten M₅ und M₆ jeweils ein "oberer" Radius R ₅ₒ beziehungsweise R₆ₒ und ein "unterer" Radius R₅ᵤ beziehungsweise R₆ᵤ vorgesehen. Der obere Radius R₅ₒ, R₆ₒ entsteht auf der einen Seite der Plattenkomponente, wobei kontinuierlich dieser Durchmesser auf den Radius R₅ᵤ, R₆ᵤ verringert wird. Es entstehen dadurch Schnittlinien 6, Schnittflächen 7 sowie schräge Flächen 7. Bei den in den Fig. 5B und 8 dargestellten Ausführungsbeispielen sind die Schnittflächen 7 auf der dem Knochen zugewandten Seite der Plattenkomponente 3 und nehmen ca. 1/3 der Tiefe der Plattenkomponente ein.

Die Anzahl der Durchgangsbohrungen ist frei zu wählen. Es wurde festgestellt, dass eine Vielzahl von Durchgangsbohrungen die Einschraubkraft der Schraube verringern. Je mehr Durchgangsbohrungen vorhanden sind, desto grösser ist zwar die Anzahl der Schnittflächen und Schnittlinien. Dennoch ist die vorhandene (aus der Draufsicht gesehen) Dicke der Schnittlinien und -flächen geringer.

Im Gegensatz zu Fig. 8 sind zum einen mehrere Durchgangsbohrungen vorgesehen und die Durchgangsbohrungen 4₁...4₈ sind konisch, wobei diese Durchgangsbohrungen sich zum Knochen K hin verjüngen.

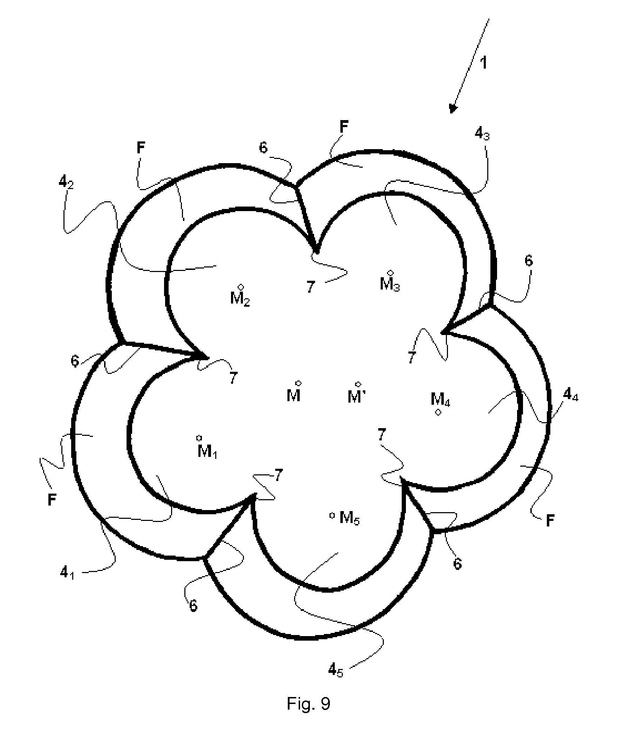

Fig. 9 zeigt eine weitere Ausführungsform, wobei die konischen Flächen im Gegensatz zu Fig. 8 asymmetrisch ausgebildet sind. Die Asymmetrie wird dadurch erreicht, dass an sich zwei zentrale Durchgangsbohrungen mit jeweils einem Mittelpunkt M, M' vorgesehen sind. Von diesen jeweiligen Mittelpunkten ausgehend erstrecken sich im unterschiedlichen Abstand die jeweiligen Mittelpunkt Mₓ (M_{1...}M₅) in unterschiedlicher Länge beziehungsweise unterschiedlichem Abstand. Dadurch entsteht in Draufsicht eine rosettenartige Ausbildung der erfindungsgemässen Durchgangsöffnung 1 eine leicht verschobene Rosettenausbildung, die unterschiedlich lange schräge Auflageflächen F bildet. Die in Draufsicht erkennbaren Schnittlinien 6 gehen dann wie bei den vorherigen Beispielen ebenfalls, mit zunehmender Tiefe in Schnittflächen über.

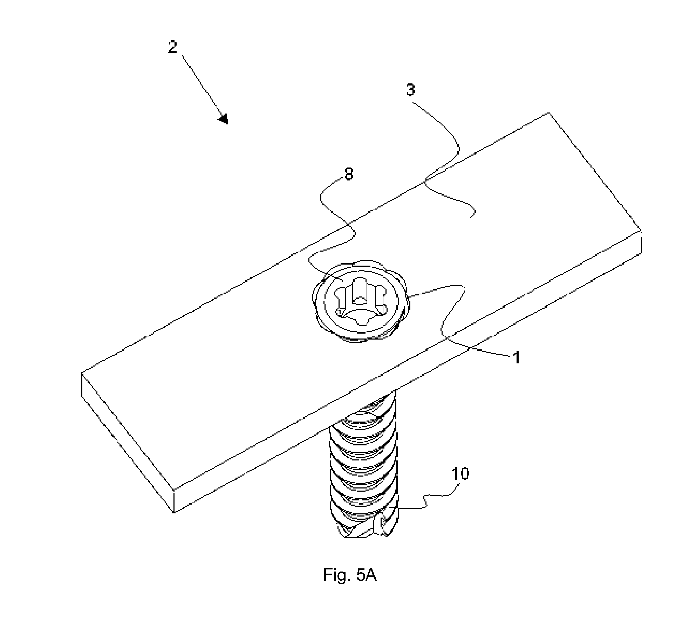

Insbesondere der in Fig. 5A bis 9 sind Ausführungen der erfindungsgemässen Durchgangsöffnung gezeigt, die besonders ausgestaltet sind. Durch die konische Ausbildung der jeweiligen Durchgangsöffnungen entsteht zunächst in Schraubrichtung eine Schnittlinie, die sich mit zunehmender Tiefe in Richtung des Knochens zu einer Schnittfläche ausbildet. Dieses ist insbesondere in Fig. 5B deutlich zu erkennen. Dies bringt den Vorteil mit sich, dass zunächst nur wenige Gewindegänge beim Einschrauben mit der Schnittlinie zusammenwirken und dort eine geringe plastische Verformung ausüben und je tiefer die Schraube hineingedreht wird, desto grösser muss die Interaktion zwischen der Schnittlinie beziehungsweise Schnittfläche und dem Gewinde der jeweiligen Schraube sein. Dadurch wird erreicht, dass aufgrund der plastischen Umformung eine nahezu nicht lösbare Verbindung entsteht.

Alternativ zu dieser konischen Ausbildung ist auch vorgesehen, die Durchgangsbohrungen, die um die zentrale Bohrung herum angeordnet sind, jeweils in einem Winkel anzuordnen, sodass sich die Durchgangsöffnung nach unten hin ebenfalls verjüngt. Damit ist es nicht notwendig, ausschliesslich konische Bohrungen herzustellen. Es ist daher auch möglich nachdem die zentrale Bohrung hergestellt worden ist, solche Ausbildungen bereitzustellen, wie sie in Fig. 5 bis 9 gezeigt sind. Durch die schräge Anordnung der jeweiligen Bohrer zur Herstellung der Durchgangsbohrung mit jeweiligem Mittelpunkt Mₓ und Radius Rₓ, wobei x Element der natürlichen Zahlen 1 bis unendlich ist, ist es daher möglich, ein selbstschliessendes Schraubenverschlusssystem, gebildet aus Schraube und Plattenkomponente zur Anwendung im orthopädischen Bereich.

Das orthopädische Schraubenverschlusssystem stellt somit eine wesentliche Neuerung gegenüber dem Stand der Technik dar, da es ausschliesslich aus zwei Elementen besteht, aber Eigenschaften aufweist, die bisher nur mit mehreren Elementen in komplexer Bauweise bekannt ist. Durch die kleeblattähnliche Struktur der Durchgangsöffnung bestehen für die Schraube Einschraubmöglichkeiten in unterschiedlichen Winkeln. Dabei zeichnet sich das System dadurch aus, dass die Schraube gegen ungewolltes Lösen gesichert ist.

Nachstehend wird anhand eines besonderen Ausführungsbeispiels eines erfindungsgemässen Plattenimplantats, das insbesondere im Bereich der Wirbelkörper Anwendung findet, das Schraubenverschlusssystem zusammen mit dem Plattenimplantat erläutert.

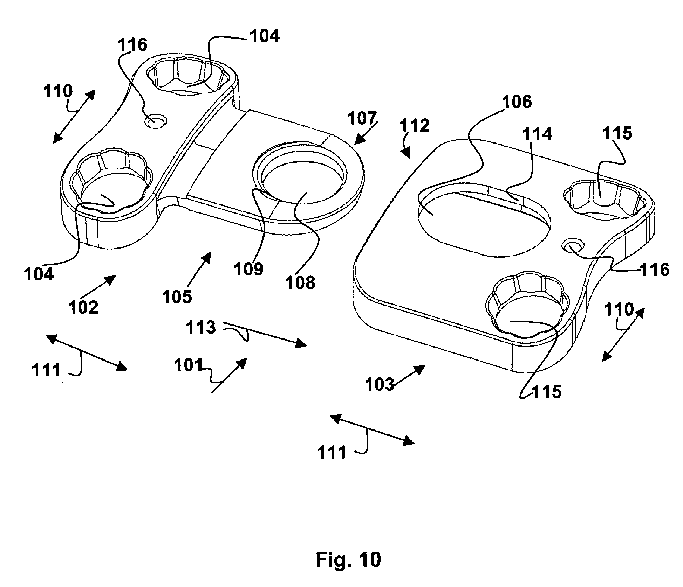

In Fig. 10 ist das Grundprinzip des erfindungsgemässen Plattenimplantats 101 dargestellt. Dieses Plattenimplantat 101 besteht aus einer ersten Plattenkomponente 102 und einer weiteren Plattenkomponente 103. Die beiden Plattenkomponenten 102, 103 sind unterschiedlich gestaltet.

Die erste Plattenkomponente 102 weist Durchgangsbohrungen 104 für noch nicht näher dargestellte Befestigungsschrauben auf. Ferner ist ein Verbindungsmittel 105 vorgesehen, das mit einem Aufnahmemittel 106 der weiteren zweiten Plattenkomponente 103 zusammenwirkt. Das Verbindungsmittel 105 ist zungenartig ausgestaltet und ist in seinem Querschnitt geringer ausgebildet als der übrige Teil der Plattenkomponente 102. Auch die Dicke des Verbindungsmittels 105 ist geringer als der übrige Teil der Plattenkomponente 101.

Im Bereich des freien Endes 107 des Verbindungsmittels 105 ist eine Bohrung 108 vorgesehen. Vorzugsweise weist diese Bohrung 108 auch ein Innengewinde 109 auf.

Die erste Plattenkomponente 102 ist sowohl in Richtung der Quererstreckung 110 als auch in Richtung der Längserstreckung 111 gebogen, sodass eine Anpassung an Wirbelkörper oder an Knochen erfolgen kann.

Wie bereits ausgeführt, ist das zungenartig ausgebildete Verbindungsmittel 105 auf das Zusammenwirken mit den Aufnahmeteil 106 der zweiten Plattenkomponente 103 ausgestaltet. Hierzu weist die zweite Plattenkomponente 103 als Aufnahmemittel 106 innerhalb des Grundkörpers der zweiten Plattenkomponente 103 eine Aussparung 112 auf, die derart ausgebildet ist, dass das zungenartige Verbindungsmittel 105 in Pfeilrichtung 113 in das Aufnahmemittel 106 eingeführt werden kann. Vorzugsweise ist zur exakten, nahezu spielfreien Führung eine Schwalbenschwanzführung vorgesehen. Das zungenartige Verbindungsmittel 105 ist derart ausgelegt, dass es auf der zum Wirbelkörper beziehungsweise Knochen hinweisenden Seite nicht fluchtend mit der Unterseite der Plattenkomponenten 102, 103 ausgelegt ist. Es ist ein Abstand vorgesehen, um zu vermeiden, dass bei Gleitbewegungen dieses Verbindungsmittel 105 auf der Oberfläche des Knochens beziehungsweise des Wirbelkörpers gleitet.

Die zweite Plattenkomponente 103 weist ferner eine Längsbohrung 114 auf, die im eingeschobenen Zustand der ersten Plattenkomponente 102 mit der Bohrung 108 des Verbindungsmittels 105 fluchtend ist.

Ferner weist die zweite Plattenkomponente 103 ebenfalls Durchgangsbohrungen 115 zur Aufnahme von Befestigungsschrauben, die in der Fig. 10 so nicht dargestellt werden, auf. Jede der Plattenkomponenten 102, 103 weisen Bohrungen 116 auf, die in ihrem Durchmesser wesentlich geringer sind als die übrigen Durchgangsbohrungen 104, 115. Diese Bohrungen 116 dienen dazu, um die Lage an dem Knochen oder den Wirbelkörpern der jeweiligen Plattenkomponenten 102, 103, bevor sie mit dem Befestigungsschrauben montiert werden, vorzufixieren.

Die zweite Plattenkomponente 103 ist in ihrer Form nahezu quadratisch ausgebildet und weist auch in ihrer Längserstreckung 111 als auch in der Quererstreckung 110 jeweils Krümmungsradien auf. Zusätzlich ist sie in diesen Richtungen ebenfalls biegbar, sodass sie an die Aussenkontur von Wirbelkörpern und Knochen anpassbar ist.

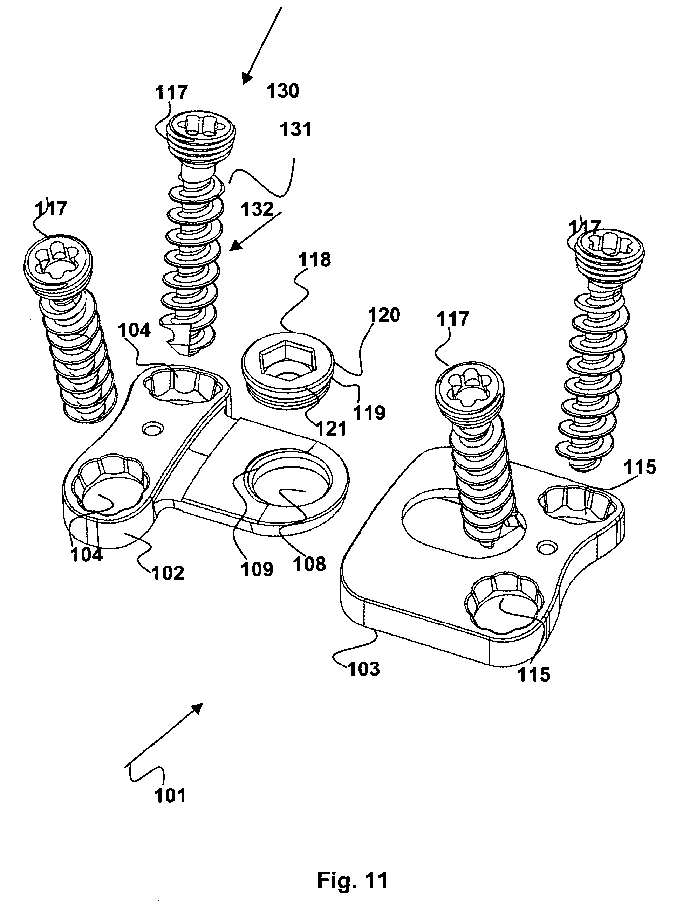

In Fig. 11 ist das Grundprinzip des Plattenimplantats 101 gemäss Fig. 10 dargestellt. Zusätzlich sind für die Durchgangsbohrungen 104 beziehungsweise 115 Befestigungsschrauben 117 dargestellt, die polyaxial in die Durchgangsbohrungen 104, 115 einführbar sind. Polyaxial bedeutet, dass diese nicht unmittelbar senkrecht zur Oberfläche der jeweiligen Plattenkomponente 102, 103 eingefügt werden müssen, sondern je nach Gegebenheit der Struktur, die sie antreffen.

Zusätzlich ist eine Klemmschraube 118 vorgesehen, die in das Gewinde 109 der Bohrung 108 eindrehbar ist. Die Klemmschraube 118 ist derart ausgebildet, dass sie ebenfalls ein Gewinde 119 aufweist, das mit dem Gewinde 109 der Bohrung 108 zusammenwirkt. Zusätzlich weist die Klemmschraube 118 im Bereich ihres Schraubenkopfes einen Wulst 120 auf, der vorzugsweise breiter gestaltet ist, als der übrige Teil des Gewindes 119. Dieser Wulst 120 weist eine nach Aussen hin verlaufende Schräge 121 auf, sodass der Durchmesser der Klemmschraube 118 von dem Gewinde 119 wegweisend grösser wird.

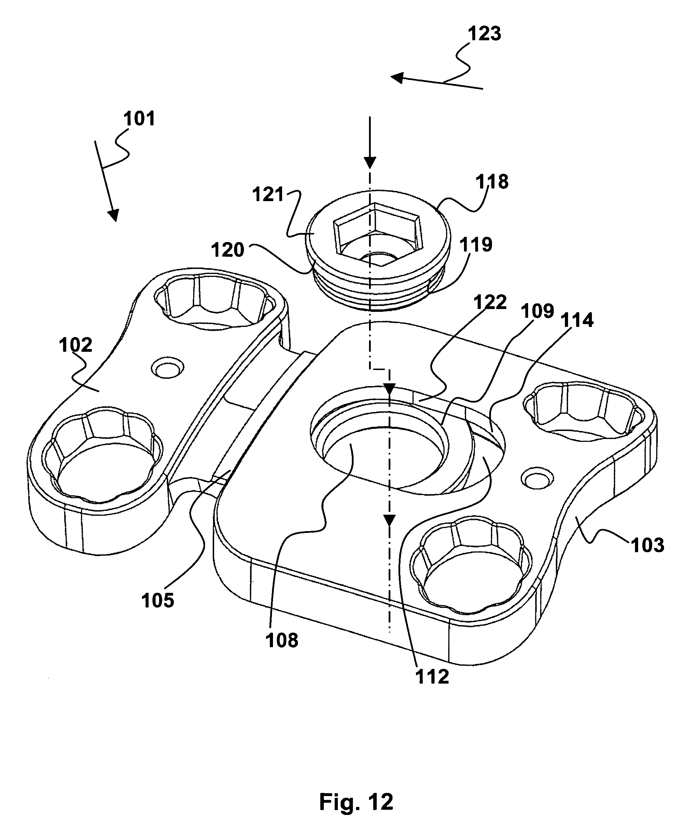

In Fig. 12 ist die Funktionsweise der Klemmschraube 118 in Verbindung mit dem Plattenimplantat 101 dargestellt. Sie wird bei zusammengefügten Plattenkomponenten 102, 103 in die Längsbohrung 114 der zweiten Plattenkomponente 103 eingefügt und das Gewinde 119 der Klemmschraube 118 tritt in Verbindung mit dem Gewinde 109 der Bohrung 108 des Verbindungsmittels 105. Der Wulst 120 tritt dabei mit den Seitenflanken 122 der Längsbohrung 114 in Verbindung derart, dass zwischen der Klemmschraube 118 und der Plattenkomponente 103 eine erste Klemmwirkung entsteht. Durch das Eindrehen der Klemmschraube 118 in die Bohrung 108 entsteht auch eine Klemmwirkung derart, dass das Verbindungsmittel 105 sich innerhalb der Aussparung 112 an die Innenseite der zweiten Plattenkomponente 103 drückt und hier eine Klemmwirkung entsteht. Es wird ausdrücklich darauf hingewiesen, dass die beiden Plattenkomponenten 102, 103 und die Klemmschraube 118 derart ausgebildet sind, dass kein Blockieren der Gleitbewegung in oder gegen die Pfeilrichtung 123 gewollt ist. Ziel ist es, eine Klemmwirkung zu erzielen, die ein Gleiten der ersten Plattenkomponenten 102 gegen die zweite Plattenkomponente 103 gegen eine definierte Kraft ermöglicht.

In der in Fig. 12 gezeigten Stellung wird die Klemmschraube 118 eingedreht, sodass die entsprechende Klemmwirkung erreicht wird. Wird durch ein so genanntes Settling eine Kraft auf das Plattenimplantat 101 ausgeübt, so bewegen sich die Plattenkomponenten 102, 103 aufeinander in Pfeilrichtung 123 zu. Die Kräfte müssen jedoch so gross sein, dass die hervorgerufene Klemmwirkung durch die Klemmschraube 118 überwunden wird. Erst dann findet eine Bewegung in Richtung Pfeilrichtung 123 statt.

Vorzugsweise bei einem anderen Ausführungsbeispiel kann auch die Längsbohrung 114 konisch ausgestaltet sein, sodass sie sich in Pfeilrichtung 123 verjüngt. Dies bedeutet, dass die Klemmkraft mit zunehmenden Settling grösser wird. Dies bedeutet auch, dass die Kräfte grösser sein müssen, um eine entsprechende Verschiebung in Pfeilrichtung 123 zu bewirken.

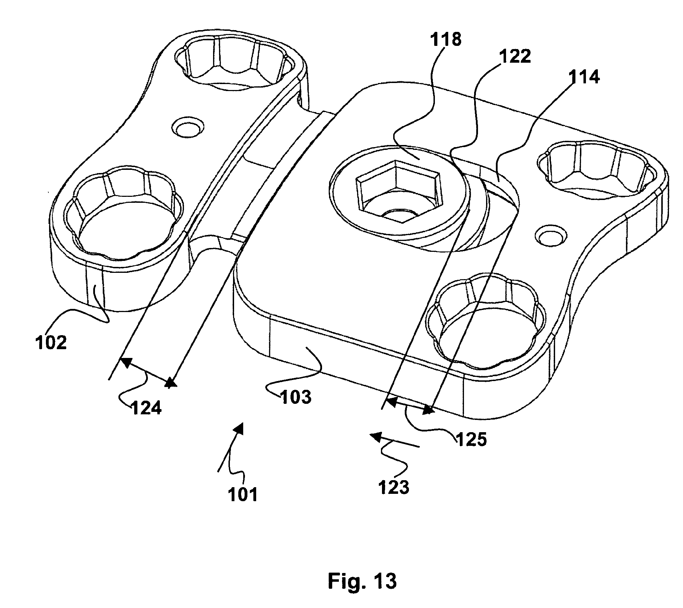

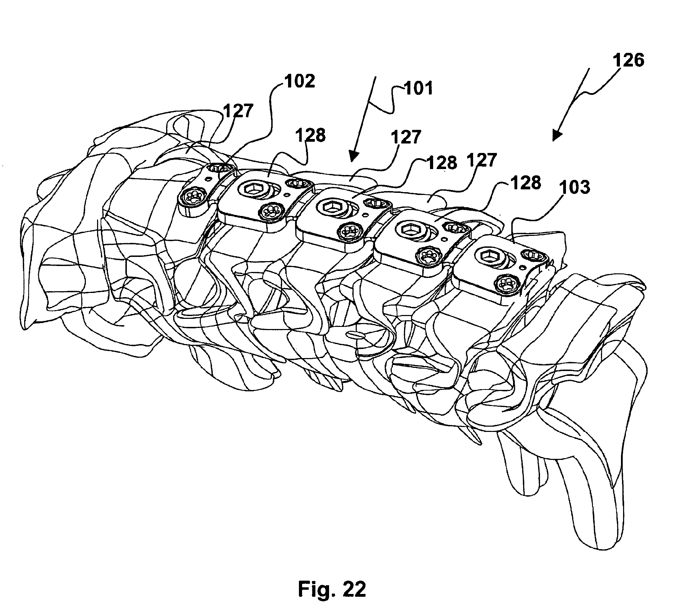

In Fig. 13 ist die in den vorherigen Fig. 10 und 11 dargestellte Grundform des Plattenimplantats 101 im montierten Zustand gezeigt. Die Klemmschraube 118 wirkt mit den Seitenflanken 122 der Längsbohrung 114 zusammen. Die Darstellung in Fig. 22 zeigt die Ausgangsstellung. Durch das Settling entsteht eine Verschiebung in Pfeilrichtung 123 und zwar die maximale Wegstrecke, die durch den Abstand 124 des einen Teils der ersten Plattenkomponente 102 zur zweiten Plattenkomponente 103 beziehungsweise durch den Abstand 125 begrenzt ist.

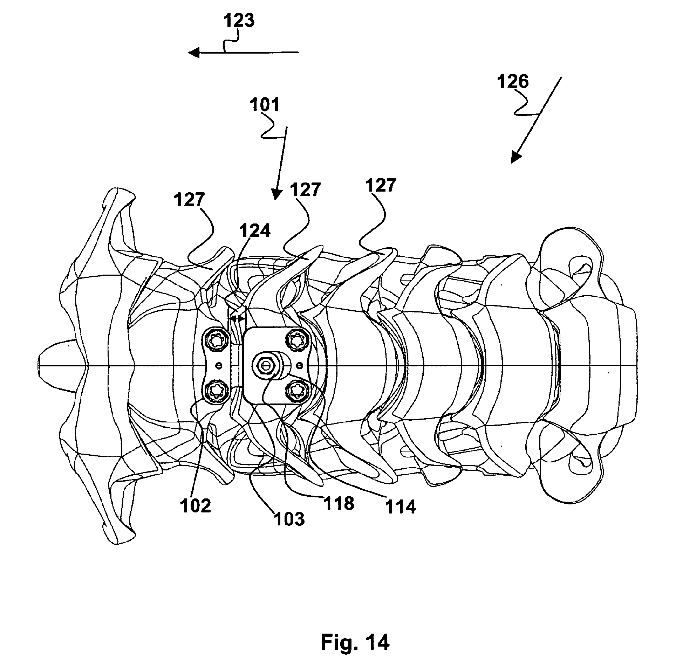

In Fig. 14 ist eine Draufsicht auf die Grundeinheit des Plattenimplantats 101 dargestellt. Die Draufsicht erfolgt auf einen Teil einer Wirbelsäule 126, die aus mehreren Wirbelkörpern 127 besteht. Bei dem hier dargestellten Ausführungsbeispiel ist die erste Plattenkomponente 102 an einem ersten Wirbelkörper 127 angeordnet, wobei die zweite Plattenkomponente 103 eine in dem nachfolgenden weiteren Wirbelkörper 127 angeordnet ist. Die beiden Plattenkomponenten 102, 103 weisen einen Abstand 124 auf, der frei gewählt worden ist, um ein Settling der beiden Wirbelkörper 127 zu kompensieren. Die Klemmschraube 118 ist derart innerhalb der Längsbohrung 114 angeordnet, dass sie auf der der ersten Plattenkomponente 102 zugewandten Seite angeordnet ist, damit eine Verschiebung der ersten Plattenkomponenten 102 gegenüber der zweiten Plattenkomponente 103 entgegen der Pfeilrichtung 123 beziehungsweise eine Verschiebung der zweiten Plattenkomponente 103 gegenüber der ersten Plattenkomponente 102 in Pfeilrichtung 123 möglich ist.

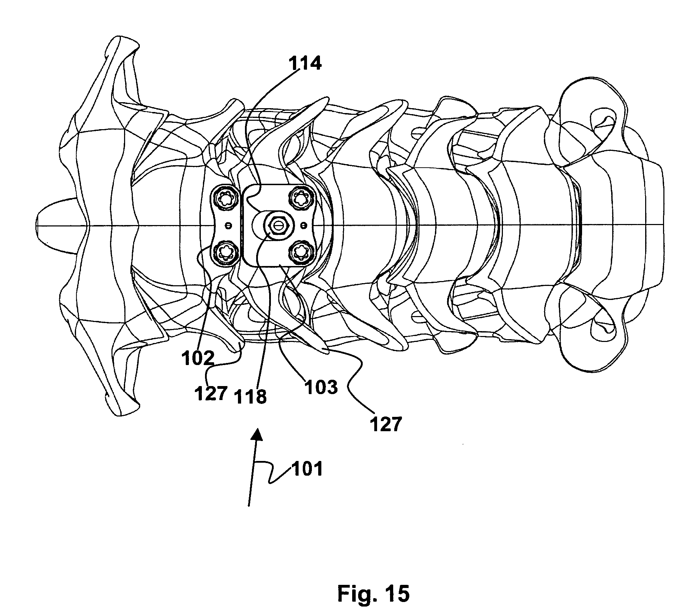

Wie in Fig. 15 dargestellt, ist ein Settling eingetreten und die beiden Plattenkomponenten 102, 103 haben sich aufeinander zubewegt. Die Klemmschraube 118 ist dabei innerhalb der Längsbohrung 114 geglitten und an ihrer Endposition angekommen. Die Wirbelkörper 127 liegen in dieser Position sehr eng beieinander, sodass weiterhin gewährleistet ist, dass der Kraftfluss nicht über das Plattenimplantat 101, sondern über die Wirbelkörper selbst erfolgt. Das Plattenimplantat 101 behält weiterhin seine Festigkeit gegenüber dem Wirbelkörper 127, da die Kräfte nicht auf die Befestigungsschrauben 117 wirken.

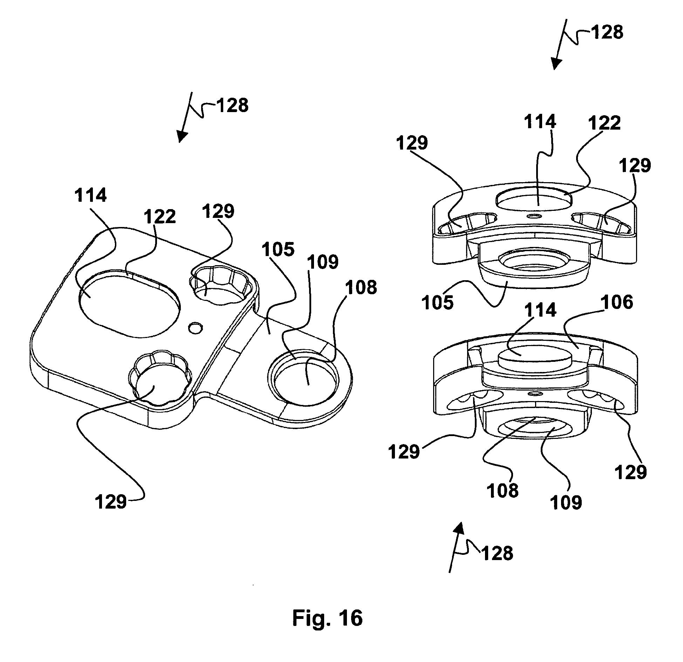

In Fig. 16 sind mehrere perspektivische Ansichten auf eine Erweiterungsplatte 128 dargestellt. Eine Erweiterungsplatte besteht ebenfalls wieder aus einem nahezu rechteckigen Grundkörper, an dessen einen Seite sich ein Verbindungsmittel 105 erstreckt. Dieses Verbindungsmittel 105 ist derart ausgestaltet, wie es bereits bei der ersten Plattenkomponente 102 gemäss Fig. 10 vorgesehen ist. Es ist zungenartig ausgebildet und weist an dessen Ende eine Bohrung 108 mit einem Innengewinde 109 auf. Ferner weist der Grundkörper 128 der Erweiterungsplatte zwei Durchgangsbohrungen 129 auf, die dazu dienen, die in der Zeichnung nicht näher dargestellten Befestigungsschrauben aufzunehmen. Ferner ist im Grundkörper eine Längsbohrung 114 vorgesehen. Diese Längsbohrung entspricht der Längsbohrung 114, wie sie bereits aus der zweiten Plattenkomponente 103 bekannt ist. Sie hat ebenfalls eine längliche Aussparung und eine definierte Seitenflanke 122. Ferner weist die Erweiterungsplatte 128 ein Aufnahmemittel 106 auf. Dieses Aufnahmemittel 106 ist derart gestaltet, wie es bereits von der zweiten Plattenkomponente 103 bekannt ist. Es dient zur Aufnahme des Verbindungsmittels 105.

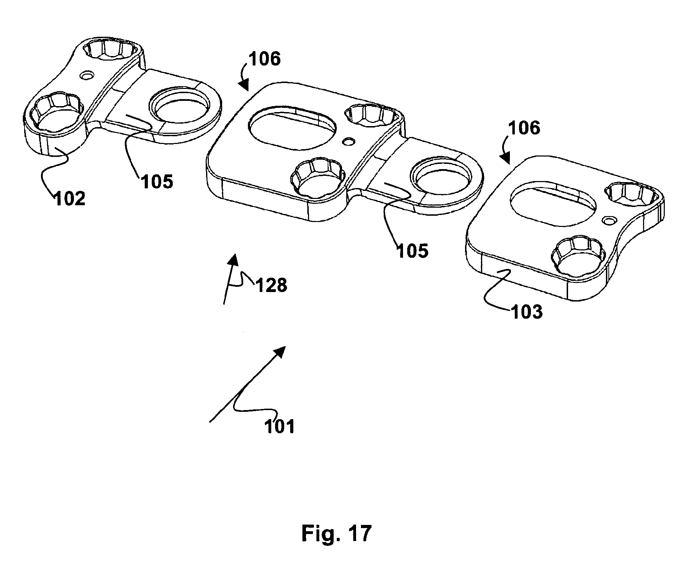

Fig. 17 zeigt in einer perspektivischen Ansicht die Anwendung einer solchen Erweiterungsplatte 128. Die Erweiterungsplatte 128 ist in der Ausbildung eines Kettengliedes zwischen die erste Plattenkomponente 102 und die zweite Plattenkomponente 103 einfügbar. Durch diese Konstellation wird ein weiteres mögliches Ausführungsbeispiel eines Plattenimplantats 101 gebildet. Je nach Bedarf können auch mehrere solcher Erweiterungsplatten 128 eingefügt werden, wobei immer gewährleistet sein muss, dass zu Beginn beziehungsweise zum Ende der "Kette" sowohl eine erste Plattenkomponente 102 als auch eine zweite Plattenkomponente 103 vorhanden sein muss. Diese beiden Plattenkomponenten 102, 103 bilden den Abschluss der "Kette".

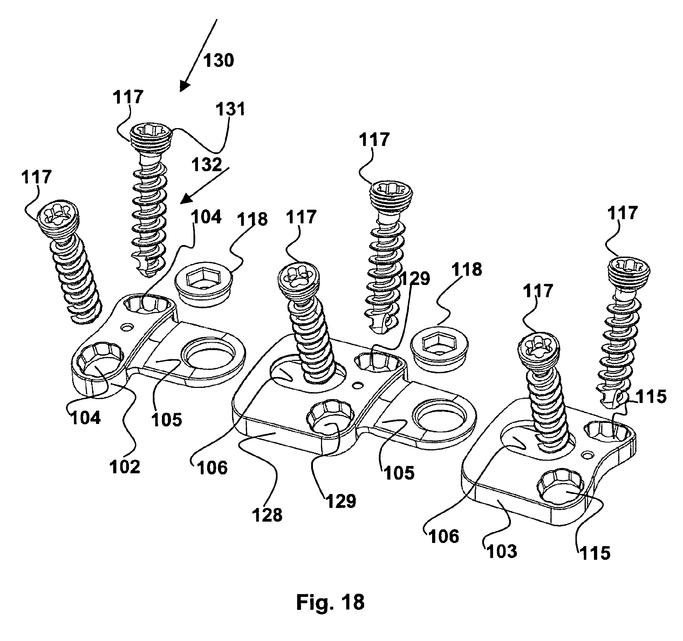

In Fig. 18 ist das gemäss Fig. 17 dargestellte Plattenimplantat 101 zusammen mit der Erweiterungsplatte 128 zusätzlich mit Befestigungsschrauben 117 und auch Klemmschrauben 118 dargestellt. Jedes der Plattenelemente weist mindestens zwei Durchgangsbohrungen 104, 115, 129 auf, in die die Befestigungsschrauben 117 polyaxial einführbar sind. Durch Zusammenwirken der Klemmschrauben 118 mit den jeweiligen Verbindungselementen 105 und dem Aufnahmemittel 106 wird die entsprechende Klemmkraft bewirkt.

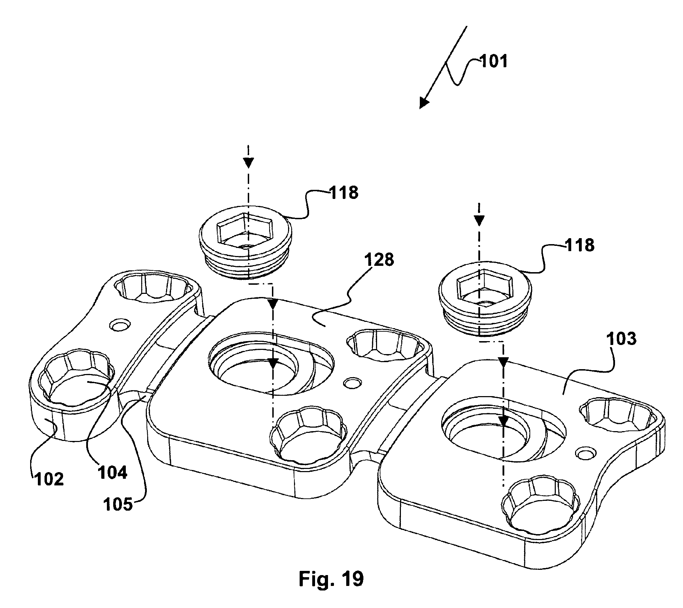

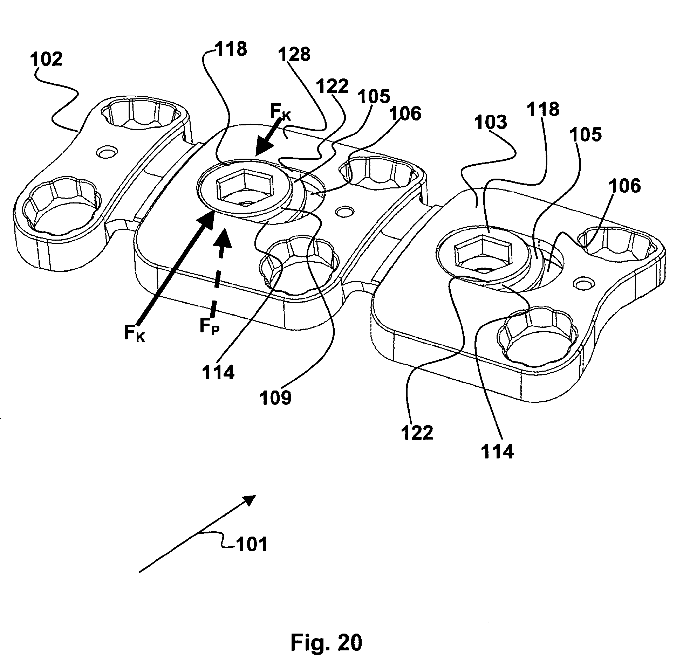

In den Fig. 19 und 20 ist die Montage und die Darstellung eines Ausführungsbeispiels eines Plattenimplantats 101, wie es in Fig. 17 und 18 bereits erläutert worden ist, dargestellt.

In der in Fig. 19 dargestellten Ansicht ist eine Gleitbewegung in uneingeschränkter Form wiedergegeben. Gegenüber Fig. 19 ist in Fig. 20 jeweils die Klemmschraube 118 eingedreht, derart, dass der Schraubenkopf der Klemmschraube 118 mit der Seitenflanke 122 der Längsbohrung 114 zusammenwirkt. Die dabei entstehenden Klemmkräfte F sind exemplarisch für alle Ausführungen der Zusammenwirkung der Klemmschraube 118 mit den jeweiligen Längsbohrungen 114 dargestellt. Zum einen wirken die Klemmkräfte F_{K}, die die Klemmschraube 118 gegenüber den Seitenflanken 122 der Längsbohrung 114 ausübt. Zum anderen wirken Klemmkräfte F_{P}, die durch das Einschrauben der Klemmschraube 118 in die Bohrung 108 des Verbindungsmittels 105 auftreten. Aufgrund dessen, dass sich der Schraubenkopf der Klemmschraube 118 an die Seitenflanken 122 der Längsbohrung anlegt, wird das Verbindungsmittel 105 an die Innenseite des Aufnahmemittels 106 herangezogen, sodass eine flächige Anlage erfolgt. Dadurch entsteht die weitere Klemmkraft F_{P}.

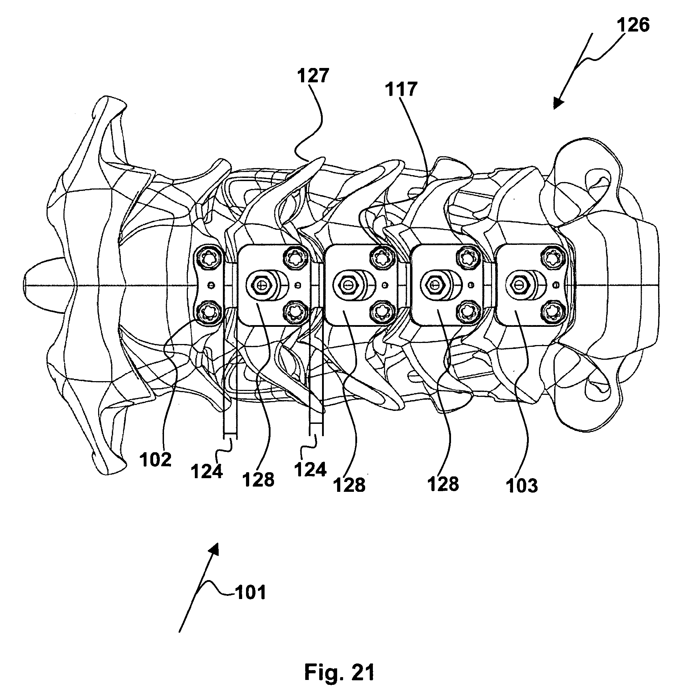

In Fig. 21 und 22 ist ein Anwendungsbeispiel dargestellt, das ein Plattenimplantat 101 zeigt, das aus der jeweiligen ersten Plattenkomponente 102 und der zweiten Plattenkomponente 103 sowie drei Erweiterungsplatten 128 besteht. Die Abstände der Erweiterungsplatten 128 sind derart gewählt, dass die Befestigungsschrauben 117 optimalen Halt in den Wirbelkörpern 127 der hier dargestellten Wirbelsäule 126 finden. Dabei ergibt sich zwischen den einzelnen Plattenkomponenten ein Abstand 124, der dafür benötigt wird, um das so zuvor erwähnte Settling auszugleichen.

Nachstehend wird die Funktionsweise der Klemmwirkung des Plattenimplantats 101 gezeigt. Insbesondere wird näher auf die Wirkung der Klemmschraube 118 im Zusammenwirken mit dem Verbindungsmittel 105 und dem Aufnahmemittel 106 eingegangen.

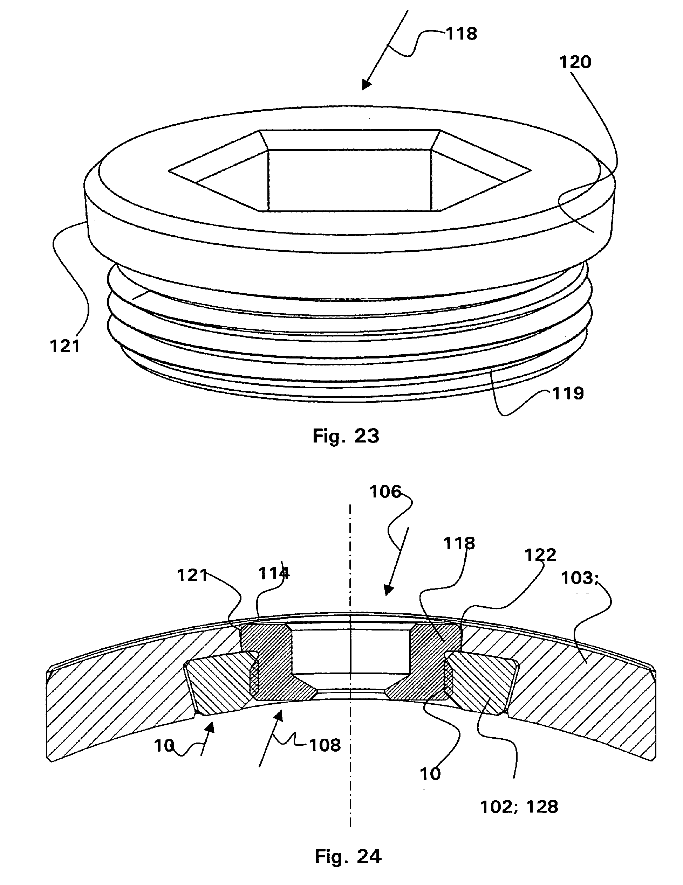

In Fig. 23 ist eine perspektivische Darstellung der Klemmschraube 118 gezeigt, wie sie in den vorherigen Figuren bereits eingesetzt worden ist. Die Klemmschraube weist im Wesentlichen zwei Bereiche auf, nämlich einen ersten Bereich eines Schraubenkopfes und einen weiteren Bereich, nämlich den Bereich eines Schraubengewindes. Der Bereich des Schraubengewindes umfasst ein Gewinde 119, das in der Regel handelsüblich ist. Es kann jedoch auch ein Feingewinde sein, damit bereits mit geringen Umdrehungen entsprechende Klemmkräfte erwirkt werden können.

Der Schraubenkopf ist speziell ausgestaltet. Er weist einen umlaufenden Wulst 120 auf, der in seinem Durchmesser zumindest gleich dem Schraubengewinde ist. Vorzugsweise ist der Schraubenkopf grösser als das Gewinde 119. Der Wulst 120 weist eine gewisse Dicke auf. Die Dicke ist derart ausgestaltet, dass sie umlaufend gleich ausgestaltet ist. Sie weist jedoch eine geringfügige Schräge 121 auf, derart, dass sich die Schräge von der dem Gewinde 119 abgewandten Seite zu der dem Gewinde 119 zugewandten Seite hin neigt.

Ferner weist die Klemmschraube 118 ein Drehmittel auf. Bei dem hier ausgeführten Ausführungsbeispiel ist das Drehmittel ein Inbus-Körper, der auf einfache Art und Weise in den Schraubenkopf eingeführt werden kann. Es entsteht nach dem Einführen ein Kraft- und Formschluss, sodass die entsprechende Drehbewegung beziehungsweise das entsprechende Drehmoment aufgebracht werden kann.

Fig. 24 zeigt einen Querschnitt durch ein Plattenimplantat 101. Der Querschnitt ist stellvertretend für alle Ausführungen des erfindungsgemässen Plattenimplantats 101 und zwar sowohl für die Grundausstattung, die aus einer ersten und zweiten Plattenkomponente 102, 103 besteht als auch für die Ausführungsbeispiele, die eine oder mehrere Erweiterungsplatten 128 aufweisen. Die Verbindung unter den einzelnen Plattenelementen ist immer die gleiche. Sie besteht in der Regel aus einem Verbindungsmittel 105, das in einem Aufnahmemittel 106 geführt ist. Vorzugsweise ist seitlich ein entsprechendes Spiel vorgesehen, sodass eine leichte Führung des Verbindungsmittels 105 innerhalb des Aufnahmemittels 106 möglich ist. Der Querschnitt gemäss Fig. 24 zeigt nun den Grundkörper entweder der zweiten Plattenkomponente 103 oder einer Erweiterungsplatte 128. In diesem Grundkörper ist bereits das Verbindungsmittel 105 eingeschoben und derart weit geführt, dass die Bohrungen 108 des Verbindungsmittels 105 mit der Längsbohrung 114 des Grundkörpers fluchtend ist. Ferner ist bereits die Klemmschraube 118 in den Grundkörper eingeführt. Die Klemmschraube 118 ist vollständig mit seinem Gewinde 119 in die Bohrung 108, das ein entsprechendes Gewinde 109 aufweist, eingedreht. Die Seitenflächen 121 der Klemmschraube 118 liegen bereits an den Seitenflanken 122 der Längsbohrung 114 an.

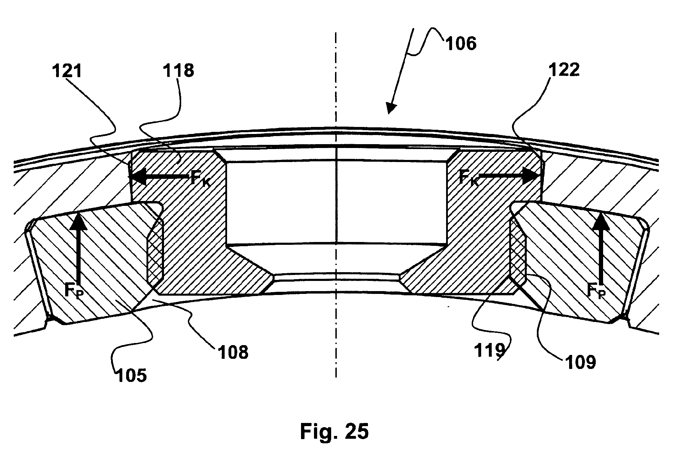

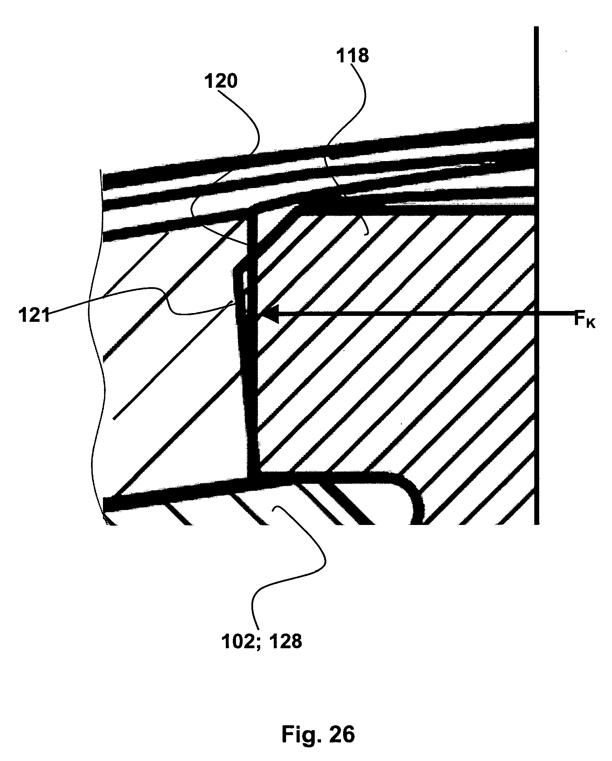

In Fig. 25 ist eine vergrösserte Darstellung der Fig. 24 gezeigt. Gegenüber Fig. 24 sind zusätzlich die Klemmkräfte eingezeichnet, die bei eingeschraubter Klemmschraube 118 wirken. Die erste Klemmkraft, die auftritt, ist diejenige, die dadurch hervorgerufen wird, dass die Klemmschraube eingedreht wird und in Verbindung mit dem Verbindungsmittel 105 steht. Durch das Einschrauben wird bewirkt, dass das Verbindungsmittel 105 unmittelbar an die Innenseite des Aufnahmemittels 106 gedrückt wird, wodurch Klemmkräfte F_{P} entstehen. Eine weitere Klemmkraft entsteht dadurch, dass die Schräge 121 der Klemmschraube 118 mit der Seitenflanke 122 der Längsbohrung 114 zusammenwirkt. Wie insbesondere aus Fig. 26 zu erkennen ist, steht die Seitenflanke auf der dem Gewinde 119 abgewandten Seite leicht in der Flucht über, sodass schon aufgrund dessen eine Klemmbeziehungsweise Keilwirkung entsteht. Dabei treten Klemmkräfte F_{K} auf. Bei gleichmässiger, symmetrischer Ausbildung der Längsbohrung sind die Klemmkräfte F_{K} nahezu gleich. Ist die Längsbohrung 114 jedoch konisch ausgestaltet, so nehmen die Klemmkräfte mit zunehmendem Erreichen des Anschlages (Wegbegrenzung der Gleitbewegung) zu.

Aufgrund der auftretenden Klemmkräfte F_{K}, F_{P} entsteht somit eine doppelte Sicherheit. Die auftretenden Klemmkräfte sind zudem in unterschiedliche Richtungen ausgerichtet, sodass diese sich nicht gegenseitig kompensieren können.

Aufgrund der Materialauswahl und der entsprechenden Auswahl der Oberflächen kann erreicht werden, dass keine Blockierung der möglichen Gleitbewegung erfolgt, sondern dass ständig gegen die ausgewählte Klemmkraft ein Gleiten des Verbindungsmittels innerhalb des Aufnahmemittels möglich ist.

Aufgrund der erfindungsgemässen Ausgestaltung des Plattenimplantats, das mindestens aus einer ersten Plattenkomponente und einer zweiten Plattenkomponente vorzugsweise aber aus zwischen der ersten Plattenkomponente und der zweiten Plattenkomponente dazwischen geschalteten Erweiterungsplatten besteht, ist ein Plattenimplantat geschaffen worden, das universell bei der Anwendung der Osteosynthese einsetzbar ist. Dieses Plattenimplantat zeichnet sich insbesondere dadurch aus, dass es das so genannte Settling, das bei der Rückbildung von versteiften Knochenelementen eintritt, ausgleicht, sodass der Kraftfluss, der normalerweise von den Knochen aufgenommen wird, auch über die Knochen weiterhin übertragen wird, sodass eine funktionelle Überbelastung des Plattenimplantats vermieden wird.

Durch die einfache Ausgestaltung und das Baukastenprinzip ist es möglich, Plattenimplantate unterschiedlicher Länge und Grösse auszuwählen. Durch einfaches Ineinanderfügen ist ein Vorfixieren ohne Probleme und ohne grösseren Aufwand möglich, sodass für den Anwender ein Anpassen, insbesondere im Bereich des Halswirbels auf sehr einfache Art und Weise vonstatten gehen kann.

Insbesondere mit der Verwendung des erfindungsgemässen Schraubenverschlusssystems ist es möglich, insbesondere Plattenimplantate, die aus mindestens zwei zueinander verschieblichen Plattenkomponenten bestehen, ein sicheres und einfach fixierbares Plattenimplantat zu schaffen. Dabei ist die Verwendung nicht davon abhängig, ob die Plattenkomponenten zueinander verriegelbar, blockierbar oder klemmbar sind.

Das Schraubenverschlusssystem ist auf alle Arten von Plattenimplantaten anwendbar, für die eine Befestigung an Knochen oder Wirbelkörpern notwendig ist.

### Bezugszeichenliste

1. Durchgangsöffnung
2. Orthopädisches Schraubenverschlusssystem
3. Plattenkomponente
4. Durchgangsbohrungen
4_{M} Zentralbohrung
4ₓ Durchgangsbohrungen
5. Abstand
6. Schnittlinien
7. Schnittflächen
8. Befestigungsschraube
9. Schraubenkopf
10. Schraubenschaft
11. Gewinde
12. Gewinde
13. Bohrung
14. Unterseite Platte
R Radius
M Mittelpunkt
K Knochen
101. Plattenimplantat
102. erste Plattenkomponente
103. zweite Plattenkomponente
104. Durchgangsöffnung
105. Verbindungsmittel
106. Aufnahmemittel
107. freies Ende des Verbindungsmittels 105
108. Bohrung
109. Innengewinde
110. Quererstreckung
111. Längserstreckung
112. Aussparung
113. Pfeilrichtung
114. Längsbohrung
115. Durchgangsbohrung
116. Bohrung
117. Befestigungsschraube
118. Klemmschraube
119. Gewinde (der Klemmschraube)
120. Wulst
121. Schräge
122. Seitenflanke
123. Pfeilrichtung
124. Abstand
125. Abstand
126. Wirbelsäule
127. Wirbelkörper
128. Erweiterungsplatte
129. Durchgangsbohrung
130. Schraubenkopf
131. Gewinde
132. Schraubenschaft

## Patentansprüche

1. Plattenimplantat zur Anwendung bei einer Osteosynthese, bestehend mindestens aus einer ersten Plattenkomponente, die Durchgangsbohrungen zur Aufnahme von Befestigungsschrauben und ein Verbindungsmittel aufweist, sowie mindestens einer zweiten Plattenkomponente, die Durchgangsbohrungen zur Aufnahme von Befestigungsschrauben und ein Aufnahmemittel zur Aufnahme eines Verbindungsmittels aufweist,
wobei jede Plattenkomponente bezüglich der anderen Plattenkomponente in einer Richtung gleiten kann und wobei die Plattenkomponenten mit einer Vorrichtung versehen sind, die ihren Gleitlauf zueinander begrenzen,
**dadurch gekennzeichnet, dass**
die Befestigungsschrauben (8,117) aus einem Schraubenschaft und einem Schraubenkopf besteht und der Schraubenschaft (10, 132) und der Schraubenkopf (9, 130) jeweils mit einem Gewinde (11, 131) versehen ist und mindestens eine Plattenkomponente (3,102,103,128) eine Durchgangsöffnung (1), gebildet aus mindestens zwei Durchgangsbohrungen (4ₓ) aufweist, wobei jede Durchgangsbohrung (4ₓ) durch einen Mittelpunkt (Mₓ) und durch einen Radius (Rₓ) bestimmt ist und die Durchgangsbohrungen (4ₓ) zueinander versetzt angeordnet sind und einander derart schneiden, dass sich Schnittlinien (6) und/oder Schnittflächen (7) bilden, die sich in die Tiefe der Durchgangsöffnung (1) erstrecken.

2. Plattenimplantat, bestehend aus mindestens einem plattenartigen Grundelement sowie mindestens eine in dem Grundelement vorgesehene Durchgangsbohrung, **dadurch gekennzeichnet, dass**
- die Plattenkomponente (3) eine Durchgangsöffnung (1), gebildet aus mindestens zwei Durchgangsbohrungen (4ₓ) aufweist, wobei jede Durchgangsbohrung (4ₓ) durch einen Mittelpunkt (Mₓ) und durch einen Radius (Rₓ) bestimmt ist,
- die Durchgangsbohrungen (4ₓ) zueinander versetzt angeordnet sind und einander derart schneiden, dass sich Schnittlinien (6) und/oder Schnittflächen (7) bilden, die sich in die Tiefe der Durchgangsöffnung (1) erstrecken.

3. Plattenimplantat nach Anspruch 1 oder 2, **dadurch gekennzeichnet, dass** die Schnittflächen (6) und/oder Schnittflächen (7) sich in Längsrichtung der Durchgangsbohrungen (4ₓ) erstrecken.

4. Plattenimplantat nach Anspruch 1, 2 oder 3, **dadurch gekennzeichnet, dass** die Mittelpunkte (Mₓ) der Durchgangsbohrungen (4ₓ) auf einem gemeinsamen Kreisbogen angeordnet sind.

5. Plattenimplantat nach einem der vorhergehenden Ansprüche, **dadurch gekennzeichnet,dass** bei drei oder mehr Durchgangsbohrungen (4ₓ) diese derart zueinander versetzt angeordnet sind, dass jede Durchgangsbohrung (4ₓ ) alle übrigen Durchgangsbohrungen (4ₓ) mindestens einmal schneidet.

6. Plattenimplantat nach mindestens einem der vorhergehenden Ansprüche, **dadurch gekennzeichnet, dass** mindestens eine Durchgangsbohrung (4ₓ) konisch ausgestaltet ist.

7. Plattenimplantat nach Anspruch 6, **dadurch gekennzeichnet, dass** sich die Durchgangsbohrungen (4ₓ) in Einschraubrichtung verjüngen.

8. Plattenimplantat nach Anspruch 6 oder 7, **dadurch gekennzeichnet, dass** in Einschraubrichtung der Durchgangsöffnung (1) zunächst Schnittlinien (6) vorgesehen, die in Schnittflächen (7) übergehen.

9. Plattenimplantat nach Anspruch 1 oder 2, **dadurch gekennzeichnet, dass** eine Durchgangsöffnung (1) durch Herstellung einer Zentralbohrung (4_{M}) mit einem Radius (R_{M}) um einen Mittelpunkt M und anschliessender weiterer Durchgangsbohrungen (4ₓ), die kleeblattartig um die Zentralbohrung (4_{M}) angeordnet sind, herstellbar ist.

10. Plattenimplantat nach mindestens einem der vorhergehenden Ansprüche, **dadurch gekennzeichnet, dass** der Schraubenkopf (8) konisch und sich zum Schraubenschaft (10) verjüngend ausgebildet ist.

11. Plattenimplantat mindestens einem der vorhergehenden Ansprüche, **dadurch gekennzeichnet, dass** die Vorrichtung eine Klemmschraube (118) umfasst, wobei die Klemmschraube (118) aus einem Schraubengewinde und einen Schraubenkopf besteht und im montierten Zustand der beiden Plattenkomponenten (102, 103) das Schraubengewinde mit dem Verbindungsmittel (105) und der Schraubenkopf mit einer Längsbohrung (114), die in der zweiten Plattenkomponente (103) vorgesehen ist zusammenwirkt, derart, dass zwischen dem Schraubenkopf und der Längsbohrung (114) und zwischen dem Verbindungsmittel und dem Aufnahmemittel (106) eine Klemmwirkung entsteht.

12. Plattenimplantat nach mindestens einem der vorhergehenden Ansprüche, **dadurch gekennzeichnet, dass** zwischen der ersten Plattenkomponente (102) und der zweiten Plattenkomponente (103) mindestens eine Erweiterungsplatte (128) vorgesehen ist, wobei die Erweiterungsplatte (128) Durchgangsbohrungen für die Aufnahme von Befestigungsschrauben (129), ein Aufnahmemittel (106) zur Aufnahme eines Verbindungsmittels (105), ein Verbindungsmittel (105) und eine Längsbohrung (114) zur Aufnahme einer weiteren Klemmschraube (118) und zur Begrenzung des Gleitlaufs aufweist.

13. Plattenimplantat nach mindestens einem der vorhergehenden Ansprüche, **dadurch gekennzeichnet, dass** die Plattenkomponenten (102, 103) und die Erweiterungsplatte (128) aus Kunststoff sind.

14. Plattenimplantat nach mindestens einem der vorgenannten Ansprüche, **dadurch gekennzeichnet, dass** die Plattenkomponenten (102, 103) und die Erweiterungsplatte (128) sowohl in ihrer Längserstreckung (111) als auch in der Quererstreckung (110) eine Krümmung aufweisen.

15. Plattenimplantat nach mindestens einem der vorgenannten Ansprüche, **dadurch gekennzeichnet, dass** die Plattenkomponenten (102, 103) und/oder die Erweiterungsplatte (128) zusätzliche Bohrungen (116) zum Fixieren des Plattenimplantats (101) aufweist.

16. Plattenimplantat nach mindestens einem der vorgenannten Ansprüche, **dadurch gekennzeichnet, dass** der Schraubenkopf der Klemmschraube (118) einen Wulst (120) aufweist, der mit den Seitenflanken (122) zusammenwirkt, derart, dass die Klemmkraft zwischen der Klemmschraube (118) und der Plattenkomponente (103) beziehungsweise Erweiterungsplatte (128) erzeugt wird.
